# EUROPEAN PATENT APPLICATION

(11) **EP 2 774 596 A1**
(43) Date of publication of application: **10.09.2014**
(21) Application number: 12845047.5
(22) Date of filing: 31.10.2012
(51) Int. Cl.: A61J 15/00

(54) **MEDICAL DILATING INSTRUMENT AND MEDICAL DILATING INSTRUMENT SET**

(30) Priority: 31.10.2011 JP 2011239229
(71) Applicant: Suzuki, Yutaka, Chuo-ku, Tokyo 1040054 (JP); Sumitomo Bakelite Company Limited, Shinagawa-ku Tokyo 140-0002 (JP)
(72) Inventor: MATSUNAMI, Hideaki, Akita-shi Akita 011-8510 (JP); IKEDA, Masao, Akita-shi Akita 011-8510 (JP); FUKUDA, Hiroyuki, Akita-shi Akita 011-8510 (JP); HARATA, Shinetsu, Akita-shi Akita 011-8510 (JP)
(74) Representative: Müller-Boré & Partner Patentanwälte PartG mbB
(86) International application number: PCT/JP2012/006968
(87) International publication number: WO 2013/065292

(57) **Abstract**

A medical dilating instrument (100) includes a needle body (10) having a pointed needle tip (11), an elongated guide portion (30) formed so that a tip (31) thereof is duller than the needle tip (11), and a trunk portion (50) having a larger diameter than the guide portion (30). A cannular needle (20) is configured by connecting the needle body (10) and the guide portion (30), and a dilator (40) is configured by connecting the guide portion (30) and the trunk portion (50). The dilator (40) includes a diameter-enlarging portion (42) that increases in diameter from a tip end side toward a base end side. The diameter-enlarging portion (42) is provided on one or both of the guide portion (30) and the trunk portion (50).

## Description

### TECHNICAL FIELD

The invention relates to a medical dilating instrument and a medical dilating instrument set.

### BACKGROUND ART

There are known dilators that dilates a small needle hole (puncture hole) formed by puncturing the body walls of a human body with a puncture needle. The dilators have a diameter-enlarging portion that gradually increases in external diameter. By inserting a tip of a dilator inside a small puncture hole formed on a body wall and by subsequently thrusting the diameter-enlarging portion into the puncture hole, the puncture hole is gradually dilated. The dilator is extracted from the body wall at a time when the puncture hole is sufficiently dilated, and medical instruments, such as a catheter, are inserted. Accordingly, a medical instrument can be percutaneously inserted into and placed inside body cavities, such as a blood vessel, an abdominal cavity, or the stomach and the intestines.

Patent Document 1 describes a cannular needle (puncture needle with a dilator) obtained by inserting a puncture needle to a cannular in which a conical tube portion that increases in diameter is formed, and connecting both of the puncture needle and the cannular. An operator indwells the cannular and extracts the puncture needle from a body wall which is punctured by the puncture needle that has protruded from the cannular. After a guide wire is inserted into this cannular, a dilator, which is larger in diameter and longer than the cannular, is thrust into the body wall along the guide wire to dilate the puncture hole (refer to FIG. 7 of Patent Document 1).

Patent Document 2 describes an entry device in which a puncture needle is inserted through a tubular dilator. The dilator is longer and larger in diameter than the puncture needle. The puncture needle has a head formed at a base end portion. After a needle tip of the puncture needle is inserted from a base end side of the dilator, the head of the puncture needle is connected to a base end portion of the dilator. A sheath including a wing is mounted around the dilator. After the sheath is thrust into the puncture hole together with the dilator, the sheath is indwelled and the puncture needle and the dilator are extracted. Then, medical instruments, such as a catheter, are inserted into this sheath.

### RELATED DOCUMENT

### PATENT DOCUMENT

[Patent Document 1] Japanese Unexamined Patent Application Publication No. S63-246178
[Patent Document 2] Published Japanese Translation No. 2010-524586 of the PCT International Publication

### DISCLOSURE OF THE INVENTION

Since blood vessels are present in a mesh shape inside a patient' s body wall, it is necessary to carefully determine the indwell position of a medical instrument. For this reason, high precision is required for the puncture position of the puncture needle. Meanwhile, a simple procedure in dilating a puncture hole (hereinafter referred to as dilating procedure) is necessary to lessen doctor's work burden granted that the procedure is safe.

Since the cannular needle of Patent Document 1 has dimensions smaller than the dilator and has good handleability, a puncture hole can be formed on a precise intended body surface position. However, there are many and complicated number of steps in the dilating procedure using the cannular needle of Patent Document 1, as there are steps to insert the guide wire into the cannular after the extraction of the puncture needle and to thrust the dilator along the guide wire. Additionally, since the body wall after the extraction of the cannular needle is self-restored and brought into close contact with the guide wire to bury the puncture hole, the procedure requires more skills, as it is necessary to insert the guide wire in a confirmed direction of the guide wire, when the dilator is inserted through the guide wire.

In the entry device of Patent Document 2, the puncture needle and the dilator are integrated together. Therefore, it is possible to puncture a body wall with the puncture needle and subsequently thrust the dilator into the puncture hole. For this reason, it is not necessary to indwell the guide wire in advance in the puncture hole before the dilator is thrust into the body wall, and the dilating procedure is simple. However, since the puncture needle and the dilator are integrated in the entry device of Patent Document 2, it is difficult for the puncture needle to puncture a precise intended body surface position. When an operator grips the vicinity of the puncture needle, the base end portion with a large moment of inertia hinders a puncture operation. Additionally, when the operator grips the base end portion, the distance from the operator hand to the tip of the puncture needle increases. Therefore, it is also difficult for the puncture needle to puncture a precise intended body surface position.

The invention has been made in view of the above problems, and object thereof is to provide a medical dilating instrument and a medical dilating instrument set capable of forming a puncture hole with high positional precision and performing the following dilating operation of the puncture hole in a simple procedure.

A medical dilating instrument of the invention includes a needle body having a pointed needle tip; an elongated guide portion formed so that a tip end thereof is duller than the needle tip; a trunk portion having a larger diameter than the guide portion; and a diameter-enlarging portion that is provided at the guide portion or the trunk portion and increases in diameter from a tip end side toward a base end side. The needle body and the guide portion are shifted from a puncture state where the needle body and the guide portion are connected to each other, the needle body is arranged along the guide portion within the proximity, and the needle tip protrudes further than the tip end of the guide portion, to a protection state where the needle tip is retreated from the tip end of the guide portion. The trunk portion is connected to a base end portion of the guide portion in the protection state.

Additionally, a medical dilating instrument set of the invention includes a needle body having a pointed needle tip; a guide portion having an elongated shape formed so that a tip end thereof is duller than the needle tip and having a mounting portion at a base end; a trunk portion having a larger diameter than the guide portion; and a diameter-enlarging portion that is provided at the guide portion or the trunk portion and increases in diameter from a tip end side toward a base end side. The needle body, the guide portion, and the trunk portion are constituted as separate bodies. The needle body or the trunk portion is mounted on the mounting portion and thereby the guide portion is arranged along the needle body within the proximity by mounting the needle tip on the mounting portion and the needle tip protrudes further than the tip end of the guide portion. The trunk portion is mounted on the mounting portion from which the needle body is separated.

In the above invention, the puncture state means a state where the needle tip protrudes further than the tip end of the guide portion and the needle tip is capable of puncturing a body wall. The protection state means a state where the needle tip is retreated from the tip end of the guide portion, and includes a state where the needle body is extracted from the guide portion, and a state where the needle body and the guide portion move relative to each other while being connected to each other and the needle body has moved further toward the base end side than the tip end of the guide portion.

According to the above invention, the trunk portion can be connected to the base end portion of the guide portion after the needle body is shifted to the protection state after the needle tip of the needle body in the puncture state has punctured the body wall. For this reason, by connecting the trunk portion to the base end portion exposed from the body wall in a state where the tip end side of the elongated guide portion is inserted inside the body wall together with the needle tip, it is possible to configure the dilator with the sufficient diameter. Hence, the needle tip can puncture a precise intended body surface position irrespective of the magnitude of the moment of inertia of the trunk portion having a larger diameter. Moreover, the dilator (diameter-enlarging portion) can be safely thrust (dilated) into a puncture hole with the guide portion as a guide in a state after the needle body is shifted to the protection state. At this time, since the dilation is started from a state where the guide portion is indwelled in the puncture hole and the self-restoration of the body wall is suppressed, the diameter-enlarging portion can be easily thrust into the puncture hole with low invasion.

In addition, the various constituent elements of the invention do not need to be individually and independently preset, and the invention allows that a plurality of constituent elements are formed as one member, one constituent element is formed by a plurality of members, a certain constituent element is a portion of another constituent element, a portion of a certain constituent element and a portion of another constituent element overlap each other, or the like.

According to the medical dilating instrument and the medical dilating instrument set of the invention, it is possible to form a puncture hole with high positional precision and perform the subsequent dilating operation of the puncture hole in a simple procedure.

### BRIEF DESCRIPTION OF THE DRAWINGS

The aforementioned objects and other objects, features, and advantages will be apparent by the preferred embodiments to be described below and the following drawings attached thereto.

FIG. 1 is an exploded front view of a medical dilating instrument related to a first embodiment of the invention.
FIG. 2 (a) is a longitudinal cross-sectional view of a cannular needle, and FIG. 2(b) is a longitudinal cross-sectional view of a dilator.
FIG. 3 is a front view of a sheath dilator.
FIGS. 4(a) to 4(d) are schematic views showing first to fourth modification examples of the vicinity of a tip end portion of a trunk portion in the sheath dilator.
FIG. 5(a) is a front view of a sheath and FIG. 5(b) is a right side view of the sheath.
FIG. 6(a) is a front view showing a state where an obturator is combined with a gastrostomy catheter.
FIG. 6 (b) is a front view showing a state where the gastrostomy catheter is extended.
FIGS. 7 (a) to 7(c) are front view showing first to third steps of forming a gastric fistula in a body wall.
FIG. 8 (a) is a front view showing a fourth step of forming the gastric fistula, FIG. 8(b) is a front view showing a state where the sheath dilator is configured, and FIGS. 8 (c) and 8 (d) are front views showing fifth to sixth steps of forming the gastric fistula.
FIGS. 9 (a) to 9 (c) are front views showing seventh to ninth steps of forming the gastric fistula.
FIG. 10 is an explanatory view showing a medical dilating instrument set of the present embodiment.
FIG. 11(a) is an exploded front view of a cannular needle of a second embodiment.
FIG. 11(b) is a front view of the cannular needle.
FIG. 12(a) is a longitudinal cross-sectional view showing a medical dilating instrument of a third embodiment, and FIG. 12(b) is a longitudinal cross-sectional view showing a cannular needle.
FIG. 13(a) is a longitudinal cross-sectional view showing a cannular needle of a fourth embodiment.
FIG. 13 (b) is an enlarged cross-sectional view of the vicinity of a needle tip.
FIG. 13(c) is a longitudinal cross-sectional view showing a needle body on which a needle tip protecting member is mounted.
FIG. 14 is an exploded view of a medical dilating instrument of a fifth embodiment.
FIG. 15 is a cross-sectional schematic view of the medical dilating instrument of the fifth embodiment.
FIG. 16 is an exploded view of a medical dilating instrument of a sixth embodiment.
FIG. 17 is a cross-sectional schematic view of the medical dilating instrument of the sixth embodiment.

### DESCRIPTION OF EMBODIMENTS

Hereinafter, embodiments of the invention will be described with reference to the drawings. In addition, in all the drawings, the same constituent elements will be designated by the same reference numerals, and the redundant description thereof will not be repeated.

### <First Embodiment>

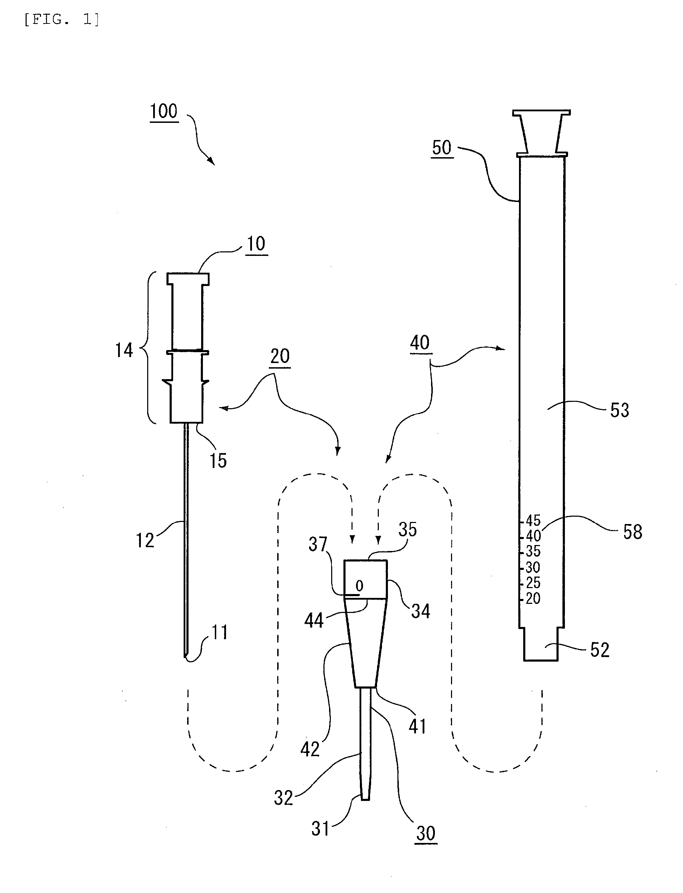

A medical dilating instrument 100, a medical dilating instrument set 200 of the present embodiment, and a method of forming a gastric fistula in a body wall using the medical dilating instrument set 200 will be described with reference to FIGS. 1 to 10. First, the outline of the medical dilating instrument 100 will be described. Hereinafter, an extending direction of a needle body 10 is referred to as a lengthy direction or axial direction of the medical dilating instrument 100, and a cross-section along the lengthy direction is referred to as a longitudinal section of the medical dilating instrument 100.

The medical dilating instrument 100 of the present embodiment includes the needle body 10 having a pointed needle tip 11, an elongated guide portion 30 formed so that a tip end 31 thereof is duller than the needle tip 11, and a trunk portion 50 having a larger diameter than the guide portion 30. As shown by a chain-line arrow in FIG. 1, a cannular needle 20 is configured by connecting the needle body 10 and the guide portion 30, and a dilator 40 is configured by connecting the guide portion 30 and the trunk portion 50. The dilator 40 includes a diameter-enlarging portion 42 that increases in diameter from a tip end side toward a base end side. The diameter-enlarging portion 42 is provided on one or both of the guide portion 30 and the trunk portion 50 (the guide portion 30 in the present embodiment).

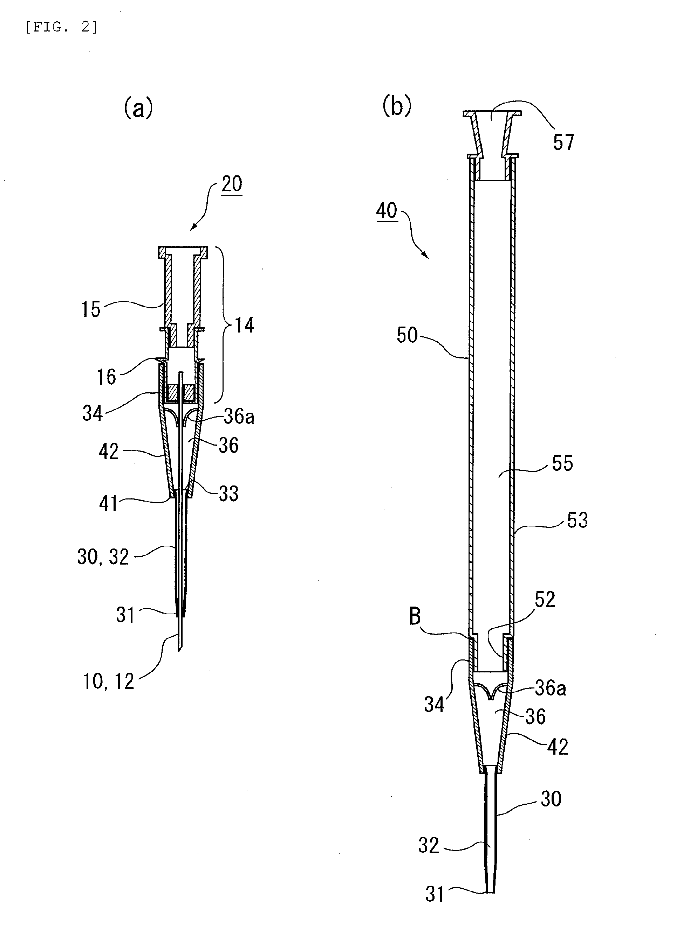

The cannular needle 20 shown in FIG. 2 (a) is in a puncture state where the needle body 10 and the guide portion 30 are connected to each other, the needle body 10 is arranged along the guide portion 30 within the proximity, and the needle tip 11 protrudes further than the tip end 31 of the guide portion 30. On the other hand, the cannular needle 20 shown in FIG. 1 is in a protection state where the needle body 10 is extracted from the guide portion 30 to the base end side, and the needle tip 11 is retreated from the tip end 31 of the guide portion 30. The cannular needle 20 is able to shift from the puncture state to the protection state. The cannular needle 20 may shift irreversibly from the puncture state toward the protection state, or may shift reversibly to the puncture state and the protection state. The cannular needle 20 of the present embodiment shifts reversibly to the puncture state and the protection state by inserting and extracting the needle body 10 with respect to the guide portion 30.

The guide portion 30 constitutes the cannular needle 20 to enter a body wall with low invasion together with a needle portion 12 at the time of the puncture of the needle body 10, while constituting the dilator 40 to function as a guide when thrusting the diameter-enlarging portion 42 and the trunk portion 50 into the body wall. The guide portion 30 of the present embodiment is constituted by a capillary portion 32 and the diameter-enlarging portion 42. Also, the cannular needle 20 is configured by combining the guide portion 30 and the needle body 10. Meanwhile, the dilator 40 is configured by combining the guide portion 30 and the trunk portion 50.

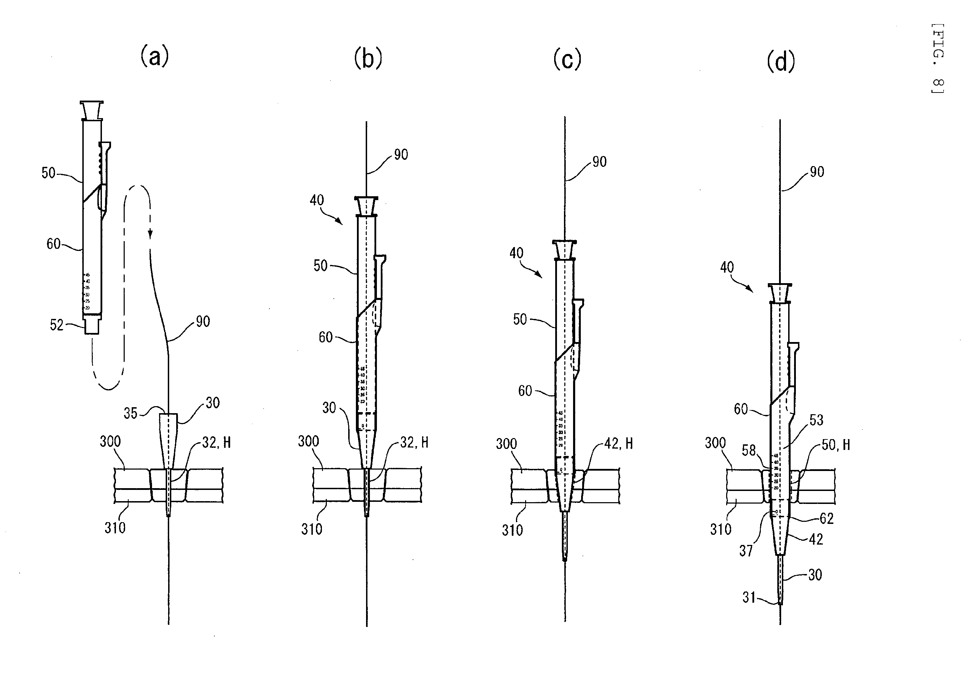

The capillary portion 32 of the guide portion 30 is a portion that is arranged along the needle portion 12 within the proximity that is connected to the needle body 10 to configure the cannular needle 20. Although the cannular needle 20 in which the tubular capillary portion 32 is circumferentially attached to the needle body 10 is illustrated in the present embodiment, the invention is not limited to this. A rod-shaped member arranged along the needle portion 12 of the needle body 10 may be used instead of the capillary portion 32. Since the medical dilating instrument 100 of the present embodiment is an instrument that can form a fistula without using a guide wire 90 (refer to FIG. 8), the medical dilating instrument 100 does not necessarily have hollow structure. Accordingly, instead of the capillary portion 32 of the guide portion 30, a rod-shaped member that is duller than the needle tip 11 and having no hole may be arranged so as to be attached to the needle portion 12.

As shown in FIG. 2 (b), the dilator 40 is configured by the trunk portion 50 being connected to a base end portion 34 of the guide portion 30 that is in the protection state by the needle body 10 being extracted from the cannular needle 20. The trunk portion 50 being connected at the guide portion 30 includes a state where both of the trunk portion and guide portion are directly joined together and a state where the guide portion 30 and the trunk portion 50 are indirectly joined together with the needle body 10 or other members being interposed therebetween.

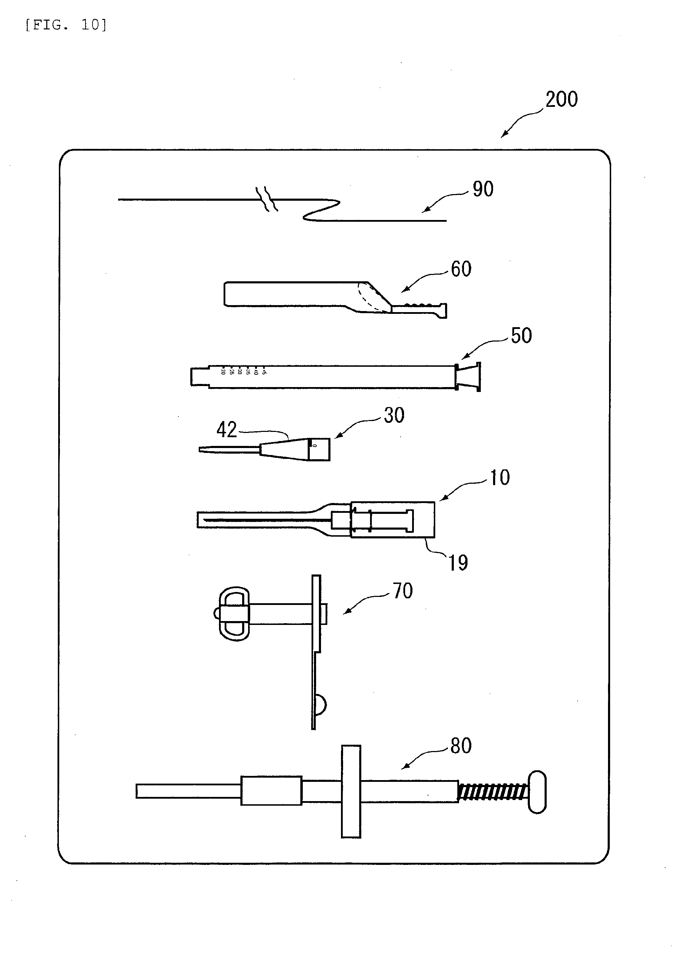

Next, the medical dilating instrument 100 will be described in detail. The medical dilating instrument 100 is a medical device for puncturing a body wall to provide a puncture hole, further dilating this puncture hole, and percutaneously inserting and placing medical instruments, such as a catheter (a cannular), a needle, or various treatment tools, within a body cavity. The medical dilating instrument 100 of the present embodiment is preferably used during the gastrostomy that forms a fistula through the abdominal wall and the stomach wall to indwell the gastrostomy catheter 70 (refer to FIG. 10).

The needle body 10 is constituted by the needle portion 12 and a head 15. A base end portion 14 of the needle body 10 is formed with the head 15. The base end portion 14 of the needle body 10 indicates a predetermined length region on a side opposite to the needle tip 11. The external diameter of the needle portion 12 is preferably 0.5 mm or more and 4 mm or less. The external diameter of the head 15 is larger than the external diameter of the needle tip 11. The needle portion 12 has an inner cavity that has hollow structure and penetrates in an axial direction. An outer periphery of the head 15 is formed with a collar portion 16 that protrudes outward in the radial direction. The head 15 has an inner cavity that penetrates in the axial direction. The inner cavities of the head 15 and the needle portion 12 communicate with each other, and are arbitrary, and enable the guide wire 90 to be inserted therethrough. A base end of the needle portion 12 is anchored inside the head 15.

As shown in FIGS. 1 and 2, a tip end portion 52 of the trunk portion 50 or the head 15 of the needle body 10 is fitted to the base end portion 34 of the guide portion 30. The tip end portion 52 of the trunk portion 50 or the head 15 of the needle body 10 is alternatively fitted to the base end portion 34 of the guide portion 30 of the present embodiment. Specifically, the tip end portion 52 of the trunk portion 50 or the head 15 of the needle body 10 is fitted to a mounting portion 35 corresponding to an opening portion on the base end side of the base end portion 34. The guide portion 30 of the present embodiment includes the capillary portion 32 and the diameter-enlarging portion 42. The guide portion 30 (the capillary portion 32 and the diameter-enlarging portion 42) has a tube shape including an inner cavity 36 with a larger diameter than the needle body 10 (needle portion 12). The inner cavity 36 of the present embodiment is a hollow portion extending over the inside of the capillary portion 32 to the inside of the diameter-enlarging portion 42. The internal diameter of the capillary portion 32 is slightly larger than the external diameter of the needle portion 12, and the needle portion 12 is slidable inside the capillary portion 32. The phrase "the guide portion 30 has an elongated shape" means that a fine-diameter portion (the capillary portion 32 in the present embodiment), which can enter the puncture hole formed by the needle tip 11 together with the needle portion 12, is formed on the tip end side of the guide portion 30. Additionally, the phrase "the trunk portion 50 has a larger diameter than the guide portion 30" means that a straight tube portion 53 of the trunk portion 50 has a large diameter than the capillary portion 32 of the guide portion 30. In addition, as in a medical dilating instrument 100 of a third embodiment to be described below, the tip end portion 52 of the trunk portion 50 may be further mounted on the base end portion 34 in a state where the head 15 of the needle body 10 is fitted to the base end portion 34 of the guide portion 30.

As shown in FIG. 2(a), the tip end 31 of the capillary portion 32 decreases in diameter in the shape of a taper. The internal diameter of the tip end 31 is equal to the external diameter of the needle portion 12, and a peripheral surface of the needle body 10 and a peripheral surface of the tip end 31 of the capillary portion 32 are smoothly continuous. Additionally, the base end 33 of the capillary portion 32 increases in diameter in the shape of a taper, and is locked to and is retained by a tip end 41 of the diameter-enlarging portion 42. The diameter-enlarging portion 42 continuously increases in diameter from the tip end 41 toward the base end portion 34. The base end portion 34 has a straight tube shape that is the same as a termination end 44 of the diameter-enlarging portion 42 on a larger diameter side.

The inner cavity 36 is provided with a lid body 36a. The lid body 36a is a member that permits insertion of the needle body 10 and regulates an air current that flows from the tip end 31 of the guide portion 30 to the base end portion 34 in the protection state. Accordingly, the lid body 36a prevents filler gas within a body cavity (stomach) from leaking out to the outside of the body through the guide portion 30 during formation of a gastric fistula. Here, the phrase "regulates an air current" means a state where the leakage of the filler gas within the body cavity has such a small flow rate that does not matter practically in addition to a state where an air current is substantially completely shielded.

The lid body 36a may be integrally formed using the same materials as that of the guide portion 30 (the capillary portion 32, the base end portion 34, or the diameter-enlarging portion 42), or may be a separate member that is used in a state being mounted on the guide portion 30. The installation position of the lid body 36a in the inner cavity 36 is not particularly limited, and may be installed inside the capillary portion 32, or may be installed inside the diameter-enlarging portion 42 or the base end portion 34. The lid body 36a of the present embodiment is a duck bill valve that has a base end portion 34 side of the guide portion 30 as an inflow side and has a tip end 31 side as an outflow side.

In addition, a turning lid that turns around an axis extending in the radial direction of the guide portion 30 to open and close the inner cavity 36, and a flap valve that reciprocally rocks similarly around an axis extending the radial direction of the guide portion 30 to open and close the inner cavity 36, may be used as the lid body 36a. Additionally, a blocking plug, which is made of soft elastic materials, such as rubber, and has an insertion hole with a larger diameter than the needle portion 12 and the guide wire inserted therethrough, may be used as the lid body 36a. The lid body 36a may be a member that, in addition to regulating an air current that flows from the tip end 31 of the guide portion 30 to the base end portion 34, regulates an air current that flows backward from the base end portion 34 to the tip end 31, as long as the needle portion 12 can be inserted through the lid body 36a in the puncture state.

In the puncture state shown in FIG. 2(a), the needle portion 12 of the needle body 10 is inserted through the lid body 36a from the base end portion 34 side toward the tip end 31 side. The lid body 36a is brought into an open state in a state where the needle portion 12 is inserted. In the protection state shown in FIG. 2(b), the needle portion 12 is extracted from the lid body 36a, and the lid body 36a is automatically brought into a closed state. The leaking of gas from the body cavity when the needle portion 12 is extracted can be prevented by providing such a lid body 36a in the guide portion 30.

As shown in FIG. 2 (b), the tip end portion 52 of the trunk portion 50 has a small diameter than the straight tube portion 53. An outer peripheral surface of the straight tube portion 53 and an outer peripheral surface of the base end portion 34 are continuous by the tip end portion 52 of the trunk portion 50 being fitted to the base end portion 34 of the guide portion 30. There is substantially no step between the base end portion 34 and the straight tube portion 53 at a connecting boundary B between the trunk portion 50 and the guide portion 30. Accordingly, external diameter increases monotonically and continuously over the capillary portion 32, the diameter-enlarging portion 42, the base end portion 34, and the straight tube portion 53 from the tip end 31 of the guide portion 30. For this reason, the puncture hole can be dilated with low invasion.

The trunk portion 50 is longer than the guide portion 30. The trunk portion 50 is formed with an insertion hole 55 for the guide wire 90. The insertion hole 55 passes through the trunk portion 50 in the direction of the tip and base ends. In the dilator 40, the insertion hole 55 communicates with the inner cavity 36 of the diameter-enlarging portion 42. A base end of the trunk portion 50 is provided with an insertion opening 57 for the guide wire 90. The trunk portion 50 is longer than the cannular needle 20, and a scale portion 58 for measuring fistula length is indicated on an outer peripheral surface of the trunk portion 50 (refer to FIG. 1). Additionally, a reference scale 37 is indicated in the vicinity of the termination end 44 of the diameter-enlarging portion 42 on the larger diameter side. A numerical value indicated by the scale portion 58 represents a distance in the lengthy direction from the reference scale 37. When the dilator 40 is thrust into the body wall and the straight tube portion 53 has entered the puncture hole, the read value of the scale portion 58 viewed at a body surface position means the depth of the dilated puncture hole (fistula).

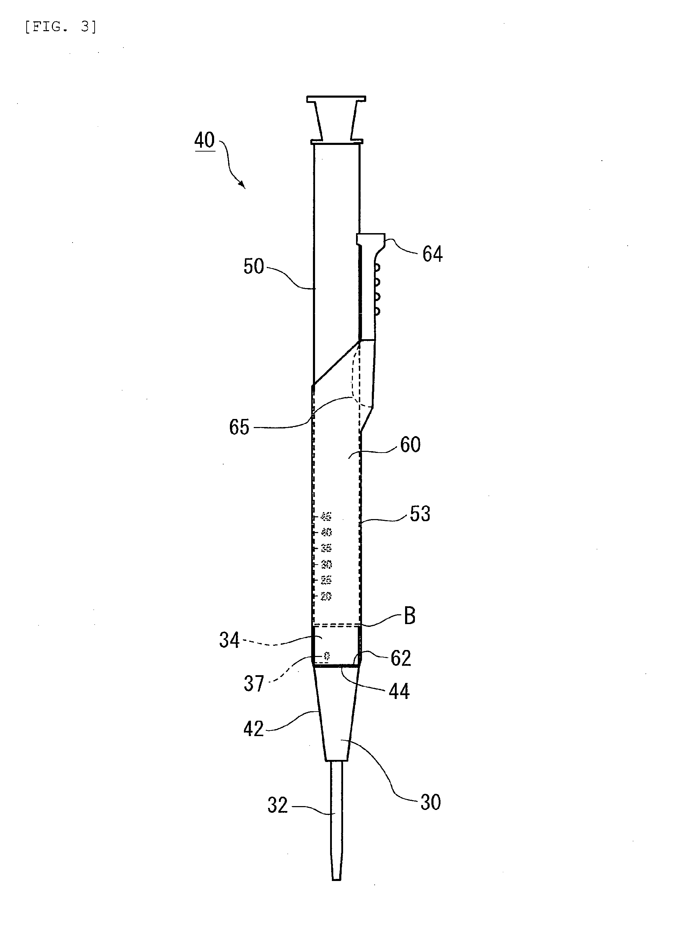

The medical dilating instrument 100 further has a sheath 60 that is used in a state being mounted around the straight tube portion 53 of the trunk portion 50 and is capable of being torn off or separable along an axis direction of the trunk portion 50. As shown in FIG. 3, the gastrostomy catheter 70 in a state where the sheath 60 is mounted around the trunk portion 50 is referred to as a sheath dilator.

A tip end of the sheath 60 is formed with a tapered portion 62 having the same diameter as the trunk portion 50 or having a slightly finer diameter than the trunk portion 50. Here, the phrase "the tapered portion 62 has a finer diameter than the trunk portion 50" means that the external diameter of the tapered portion 62 is smaller than the external diameter of the trunk portion 50. As shown in FIG. 3, the sheath 60 of the present embodiment covers the boundary (connecting boundary B) between the base end portion 34 of the guide portion 30 and the trunk portion 50. The tapered portion 62 of the sheath 60 is located at the reference scale 37 or the termination end 44 of the diameter-enlarging portion 42 on the larger diameter side, or in the vicinity thereof. However, the tapered portion 62 of the sheath 60 may reach an intermediate portion of the diameter-enlarging portion 42. An outer peripheral surface of the tapered portion 62 of the sheath 60 and an outer peripheral surface of the diameter-enlarging portion 42 are continuous. For this reason, when the dilator 40 is thrust into the puncture hole (fistula), the sheath 60 enters the fistula smoothly.

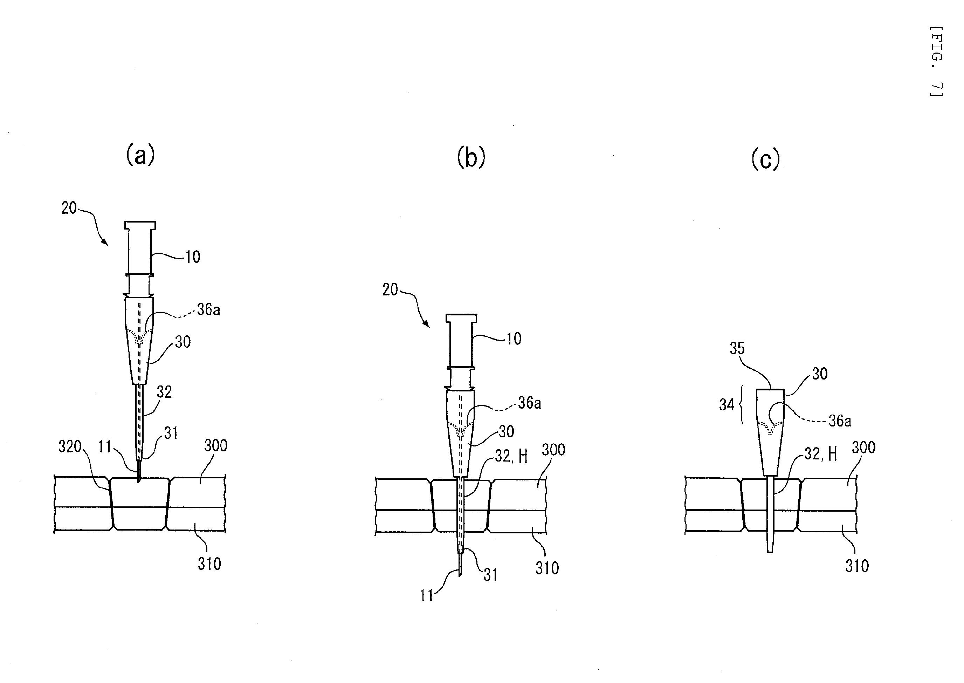

In a gastric fistula forming method to be described below, it is preferable that the step in the radial direction and a gap in the axial direction in the connecting boundary B between the guide portion 30 and the trunk portion 50 be small. Accordingly, during the dilation of thrusting the dilator 40 into the body wall or when the sheath 60 is indwelled in the puncture hole H (refer to FIG. 7) and the dilator 40 is extracted, these procedures can be performed with low invasion. Similarly, it is preferable that the step in the radial direction between the tapered portion 62 of the sheath 60 and the trunk portion 50 or the diameter-enlarging portion 42 be small.

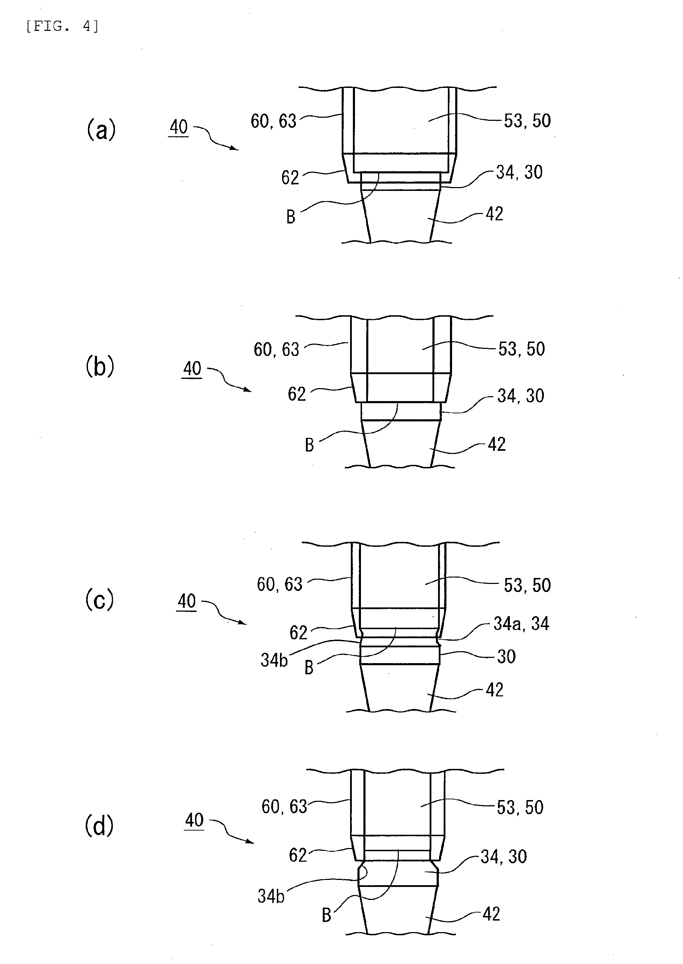

FIGS. 4(a) to FIG. 4(d) are schematic views showing first to fourth modification examples near the tip end of the trunk portion 50 in the dilator 40. In these drawings, the trunk portions 50 viewed through the transparent sheaths 60 are shown in solid lines. According to these modification examples, the tapered portion 62 is kept from interfering with a peripheral wall of the puncture hole H (refer to FIG. 7) during dilation.

A step in a diameter-decreasing direction, that is, a step that decreases in diameter toward the tip end side, is present between the straight tube portion 53 of the trunk portion 50 of a first modification example of FIG. 4(a), and the base end portion 34 of the guide portion 30. The base end portion 34 has a smaller diameter than the straight tube portion 53. The tapered portion 62 of the sheath 60 is located at the base end portion 34. Since the thickness of the tapered portion 62 of the sheath 60 is present inside the step between the base end portion 34 and the straight tube portion 53, the tapered portion 62 of the sheath 60 is kept from interfering with the peripheral wall of the puncture hole H.

A step in a diameter-increasing direction, that is, a step that increases in diameter toward the tip end side, is present between the straight tube portion 53 of a second modification example of FIG. 4(b), and the base end portion 34. The base end portion 34 has a larger diameter than the straight tube portion 53. The tapered portion 62 of the sheath 60 is in contact with a lower end of the straight tube portion 53, that is, a base end side of the base end portion 34. When the dilator 40 is thrust into the body wall, the thickness of at least a portion of the tapered portions 62 of the sheath 60 is accommodated in the step between the base end portion 34 and the straight tube portion 53. For this reason, the tapered portion 62 of the sheath 60 is kept from interfering with the peripheral wall of the puncture hole H.

The base end portion 34 of a third modification example of FIG. 4(c) has a small-diameter constricted portion 34a formed at an intermediate portion in the axial direction (a vertical direction of this drawing). The external diameter decreases smoothly from a lower end of the straight tube portion 53 to the constricted portion 34a. The tapered portion 62 of the sheath 60 is located at the constricted portion 34a. For this reason, the peripheral wall of the puncture hole H dilated by the diameter-enlarging portion 42 and the tapered portion 62 are kept from interfering with each other. Additionally, an inclined surface 34b is formed continuously with a tip end side of the constricted portion 34a. The inclined surface 34b has an inverted tapered shape that is dilated toward the tip end side. By forming this inclined surface 34b on the base end portion 34, the guide portion 30 does not interfere with the tapered portion 62 when the sheath 60 is indwelled in the puncture hole (fistula) H and the dilator 40 is extracted from the sheath 60 (refer to FIG. 8(d)).

In the base end portion 34 of a fourth modification example of FIG. 4(d), an inclined surface 34b is provided at the step in the diameter-increasing direction between the straight tube portion 53 and the base end portion 34 in the second modification example (refer to FIG. 4 (b)). The inclined surface 34b has an inverted tapered shape that is dilated toward the tip end side. The tapered portion 62 of the sheath 60 is located on the inclined surface 34b. Since the tapered portion 62 of the sheath 60 is concealed behind the inclined surface 34b, the tapered portion 62 of the sheath 60 is kept from interfering with the peripheral wall of the puncture hole H. As the tapered portion 62 of the sheath 60 is located on the inclined surface 34b, similar to the third modification example, the guide portion 30 does not interfere with the tapered portion 62 when the sheath 60 is indwelled in the puncture hole (fistula) H and the dilator 40 is extracted from the sheath 60.

As in the dilators 40 of the second to fourth modification examples, a fine-diameter region that is smaller than the maximum external diameter of the diameter-enlarging portion 42 may be formed at the connecting boundary B between the guide portion 30 and the trunk portion 50 or its tip end side, and the sheath 60 may be mounted on the trunk portion 50 so that the tapered portion 62 is located in the fine-diameter region.

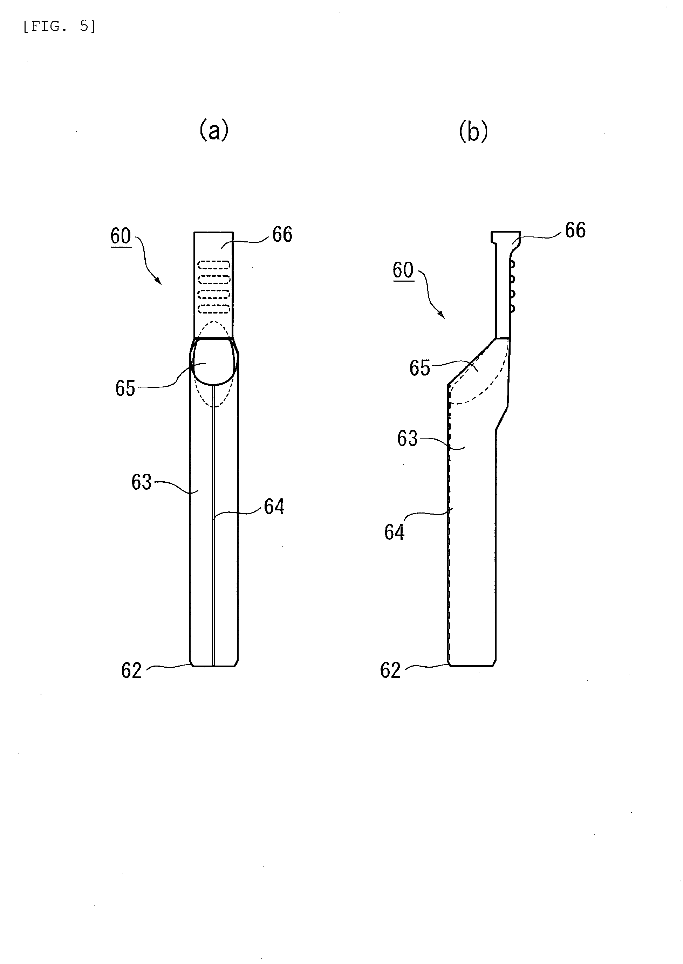

As shown in FIGS. 5 (a) and 5 (b), the sheath 60 includes a gripper 66, a valve portion 65, and a tubular portion 63. The tubular portion 63 is made of a transparent resin material. The tubular portion 63 is formed with a half-cut groove 64. The half-cut groove 64 is formed over the total length of the tubular portion 63 in the lengthy direction. The valve portion 65 is in contact with an inner peripheral surface of the tubular portion 63 on the base end side. A base end side of the valve portion 65 is fixed to the gripper 66, and a tip end side thereof is rockable with respect to the gripper 66. Accordingly, an opening of the tubular portion 63 on the base end side is openable and closable and is airtightly closed.

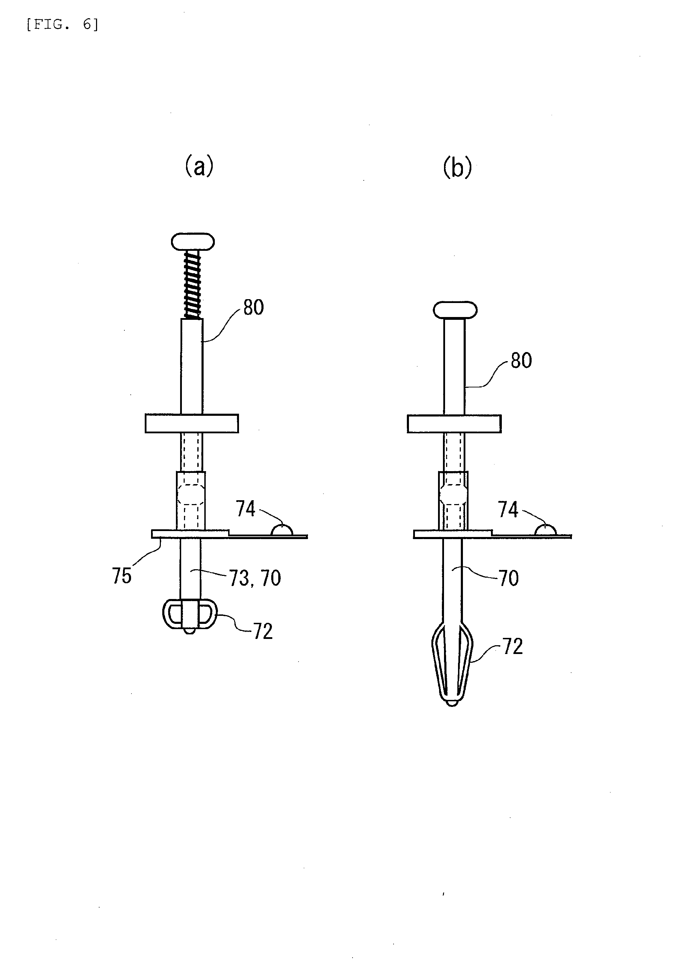

The medical dilating instrument 100 of the present embodiment is used together with the gastrostomy catheter 70 and an obturator 80 (refer to FIGS. 6(a) and 6(b)). The gastrostomy catheter 70 is also referred to a gastric-fistula button. The gastrostomy catheter 70 is a catheter that is indwelled from a body surface to the inside of the stomach wall, and is an instrument that enables enteral nutrition intake to be performed by the percutaneous endoscopic gastrostomy (PEG). A tip end of the gastrostomy catheter 70 is formed with a flexible intracorporeal indwelling portion 72. The obturator 80 of the present embodiment is an instrument that is inserted through the gastrostomy catheter 70 to extend the intracorporeal indwelling portion 72 to thereby reduce the diameter of a forefront portion of the intracorporeal indwelling portion 72 to a fine diameter smaller than the sheath 60. FIG. 6(a) shows a state where the obturator 80 is mounted on the inside of the gastrostomy catheter 70. FIG. 6(b) shows a state where the obturator 80 is pressed toward the tip end side (downward in this drawing) with respect to the gastrostomy catheter 70 and thereby the tip end of the obturator 80 extends the intracorporeal indwelling portion 72 downward. The intracorporeal indwelling portion 72 is of a non-balloon type (malecot type) as an example. The gastrostomy catheter 70 has a straight tube portion 73 that is inserted into the fistula, an abutting plate 75 formed in the upper portion of the straight tube portion 73, and a blocking plug 74 that blocks an opening of the straight tube portion 73 on the base end side.

A method (gastric fistula forming method) for forming the fistula in the body wall by the medical dilating instrument 100 of the present embodiment to indwell the gastrostomy catheter 70 will be described with reference to FIGS. 7, 8, and 9.

First, as a preliminary step of gastric fistula formation, as are shown in FIG. 7(a), an abdominal wall 300 and a stomach wall 310 are fixed with a suture 320 so as to surround the periphery of a predetermined forming position of a puncture hole. Additionally, the cannular needle 20 is configured by connecting the guide portion 30 and the needle body 10. The needle tip 11 of the needle body 10 is in the puncture state where the needle tip has protruded from the tip end 31 of the guide portion 30. Next, in a first step of the gastric fistula formation, the needle body 10 is perpendicularly bumped against the abdominal wall 300, and the abdominal wall 300 is punctured by the needle tip 11. The cannular needle 20 of the present embodiment has a total length that is approximately equal to the needle body 10 shorter than the trunk portion 50 excluding the trunk portion 50. For this reason, the needle tip 11 can puncture precise intended predetermined forming position of the puncture hole.

In a second step shown in FIG. 7(b), the cannular needle 20 is further pushed in, and the tip end 31 of the guide portion 30 is inserted inside the puncture hole H, following the needle tip 11. Accordingly, the puncture hole H is dilated to the external diameter of the capillary portion 32 of the guide portion 30. The tip end 31 of the guide portion 30 preferably protrudes from a lower surface (inner surface) of the stomach wall 310. In other words, the capillary portion 32 of the guide portion 30 is preferably longer than the total thickness of the abdominal wall 300 and the stomach wall 310.

In a third step shown in FIG. 7 (c), the needle body 10 (not shown in this drawing) is extracted from the guide portion 30 with the guide portion 30 being indwelled in the puncture hole H, and the cannular needle 20 (the same) is shifted to the protection state. Accordingly, the mounting portion 35 of the guide portion 30 opens. The mounting portion 35 is formed at the base end portion 34 of the guide portion 30 exposed above the puncture hole H.

The lid body 36a is installed in the inner cavity 36 of the guide portion 30. The lid body 36a of the present embodiment is a duck bill valve that unidirectionally regulates an air current that flows from the tip end 31 of the guide portion 30 to the base end portion 34. In the puncture state of FIGS. 7 (a) and 7 (b), the needle portion 12 of the needle body 10 is inserted through the lid body 36a. In the protection state of the present embodiment shown in FIG. 7(c), the needle portion 12 is extracted from the lid body 36a. Accordingly, the lid body 36a closes the inner cavity 36. The inside of the body cavity (stomach) is filled with gas with higher pressure than the atmospheric pressure. As this filler gas expands the stomach in the shape of a bag, the visual field of an endoscope is maintained in a widely stable state. The lid body 36a prevents the filler gas from leaking out through the guide portion 30 in the third step and its subsequent steps. As the lid body 36a closes the inner cavity 36 in a dilating step to be described below, the filler gas is kept from leaking out from the body cavity in the dilating step.

In a fourth step shown in FIG. 8(a), the tip end portion 52 of the trunk portion 50 mounted with the sheath 60 is mounted on the mounting portion 35 of the guide portion 30. The sheath 60 is slidable with respect to the trunk portion 50. In a state where the tip end portion 52 of the trunk portion 50 is exposed to the tip end side from the sheath 60, this tip end portion 52 is mounted on the mounting portion 35. Thereafter, it is preferable to make the sheath 60 slide to the tip end side to match the tapered portion 62 with the reference scale 37, the termination end 44 of the diameter-enlarging portion 42 on the larger diameter side, or the vicinity thereof. At this time, the guide wire 90 may be arbitrarily used. Specifically, after the guide wire 90 is inserted inside the opening (mounting portion 35) of the base end of the guide portion 30 indwelled in the puncture hole H, the trunk portion 50 is mounted on the mounting portion 35 along the guide wire 90. The guide wire 90 is inserted through the lid body 36a, and protrudes further than the tip end 31 of the guide portion 30. The guide wire 90 is sufficiently thin, and the filler gas within the stomach does not leak out significantly from a gap between the lid body 36a and the guide wire 90.

However, the tip end portion 52 of the trunk portion 50 is mounted on the mounting portion 35 without using the guide wire 90 in the gastric fistula formation of the present embodiment. FIG. 8 (b) shows a state where the trunk portion 50 and the guide portion 30 are combined together to configure the dilator 40. The sheath 60 is closely mounted around the trunk portion 50. At this time, as shown in this drawing, the tip end 31 of the dilator 40 (guide portion 30) protrudes from the lower surface (inner surface) of the stomach wall 310, the puncture hole H with the external diameter of the capillary portion 32 is formed to penetrate from the abdominal wall 300 to the stomach wall 310. As the puncture hole H reaching the inside of the body cavity (stomach) is formed in advance, the dilator 40 can be smoothly inserted inside the body wall (the abdominal wall 300 and the stomach wall 310) in fifth to sixth steps to be described below.

FIGS. 8(c) and 8(d) show the dilating steps. The capillary portion 32 of the guide portion 30 is indwelled in the puncture hole H when the dilation is started. For this reason, the dilation is started in a state where the self-restoration of the abdominal wall 300 and the stomach wall 310 is suppressed.

In a fifth step shown in FIG. 8(c), the trunk portion 50 is gripped and the dilator 40 is thrust into the puncture hole H. The diameter-enlarging portion 42 enters the abdominal wall 300 and the stomach wall 310 to dilate the puncture hole H.

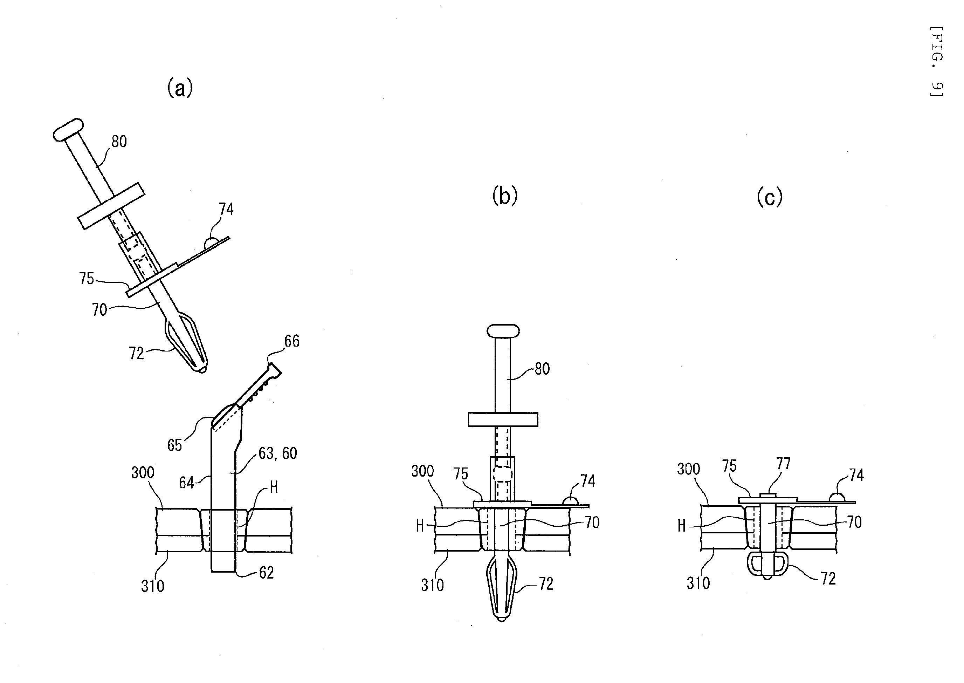

In a sixth step shown in FIG. 8 (d), the trunk portion 50 is thrust into the puncture hole H, following the diameter-enlarging portion 42. The diameter-enlarging portion 42 reaches the inside of the body cavity (stomach) below the stomach wall 310. The tapered portion 62 of the sheath 60 reaches a portion below the stomach wall 310 together with the diameter-enlarging portion 42. The diameter of the puncture hole H is equal to the maximum external diameter of the diameter-enlarging portion 42, that is, the external diameter of the straight tube portion 53 of the trunk portion 50. It is preferable to indwell the endoscope (not shown) within the body cavity (within the stomach) and optically observe the vicinity of the puncture hole H. In the sixth step, by endoscopically confirming that the reference scale 37 has reached the inside of the stomach through the lower surface (the anterior wall of the stomach) of the stomach wall 310, it is confirmed that the puncture hole H is dilated to the same diameter as the external diameter of the trunk portion 50 over its total length. Additionally, subsequently, the reference scale 37 is matched with the anterior wall of the stomach, for example, by retreating the dilator 40 slightly. The total thickness of the abdominal wall 300 and the stomach wall 310 is measured from the read value of the scale portion 58 viewed at the body surface (the upper surface of the abdominal wall 300) in that state. The effective length size of the gastrostomy catheter 70 (refer to FIG. 9) is determined based on this read value. It is preferable to select an effective length size about 5 to 10 mm longer than the read value. Thereafter, the sheath 60 is indwelled in the puncture hole H, and the dilator 40 and the guide wire 90 are extracted. The puncture hole (fistula) H is formed from the above. In addition, in the fifth step or sixth step, the guide wire 90 may be extracted prior to the dilator 40.

Subsequently, in a seventh step shown in FIG. 9(a), the gastrostomy catheter 70 is inserted into the sheath 60 indwelled in the dilated puncture hole H. In the sheath 60 from which the dilator 40 is extracted, the opening of the tubular portion 63 on the base end side is closed by the valve portion 65. The obturator 80 is inserted into the gastrostomy catheter 70, and the intracorporeal indwelling portion 72 is extended to the tip end side and decreased in diameter. If the intracorporeal indwelling portion 72 is inserted into the sheath 60, the half-cut groove 64 of the tubular portion 63 breaks from the base end side, and the sheath 60 is torn off. The gastrostomy catheter 70 is inserted inside the puncture hole H, tearing off the sheath 60. The gastrostomy catheter 70 is inserted inside the puncture hole (fistula) H until the intracorporeal indwelling portion 72 is confirmed within the stomach (under the endoscope). The abutting plate 75 is larger than the puncture hole H after the dilation.

In an eighth step shown in FIG. 9(b), the gastrostomy catheter 70 and the obturator 80 are inserted inside the puncture hole (fistula) H, and the torn-off sheath 60 (not shown in this drawing) is extracted from the puncture hole (fistula) H.

In a ninth step shown in FIG. 9(c), release of the extension of the obturator 80 (not shown in this drawing) is performed, and the obturator 80 is then extracted from the gastrostomy catheter 70. Accordingly, the intracorporeal indwelling portion 72 increases in diameter to its natural state. The intracorporeal indwelling portion 72 in the natural state has a larger diameter than the puncture hole H after the dilation. As the body wall (the abdominal wall 300 and the stomach wall 310) is pinched by the abutting plate 75 and the intracorporeal indwelling portion 72, the gastrostomy catheter 70 is indwelled without separating from the puncture hole H. This completes the indwelling of the gastrostomy catheter. Except for a case where instruments, such as a tube, are inserted into the gastrostomy catheter 70, the opening 77 of the straight tube portion 73 on the base end side is blocked by the blocking plug 74.

FIG. 10 is a schematic view showing a medical dilating instrument set 200 used for the above-described gastric fistula forming method. The left side of this drawing corresponds the tip end sides of the respective instruments. The medical dilating instrument set 200 includes the needle body 10 having the pointed needle tip 11, the guide portion 30 having an elongated shape formed so that the tip end thereof is duller than the needle tip 11, and having the mounting portion 35 at the base end, the trunk portion 50 having a larger diameter than the guide portion 30, and the diameter-enlarging portion 42 that is provided at the guide portion 30 or the trunk portion 50 (at the guide portion 30 in the present embodiment) and increases in diameter from the tip end side toward the base end side. The needle body 10, the guide portion 30, and the trunk portion 50 are configured as separate bodies. The needle body 10 or the trunk portion 50 is alternatively mounted on the mounting portion 35, the guide portion 30 is arranged along the needle body 10 within the proximity by the needle body 10 is mounted on the mounting portion 35 and the needle tip 11 protrudes further than the tip end 31 of the guide portion 30, and the trunk portion 50 is mounted on the mounting portion 35 of the guide portion 30 from witch the needle body 10 (refer to FIG. 2) is separated.

Also, the medical dilating instrument set 200 of the present embodiment includes the sheath 60 that is used in a state being mounted around the trunk portion 50 and is capable of being torn off along the axis direction of the trunk portion 50, the gastrostomy catheter 70, and the obturator 80. That is, the medical dilating instrument set 200 of the present embodiment is a gastrostomy set for indwelling the gastrostomy catheter in the body wall.

In the medical dilating instrument set 200, the needle body 10 is preferably housed in a protective case 19. Additionally, the guide wire 90 may be arbitrarily included in the medical dilating instrument set 200. When the guide wire 90 is included, it is preferable to house the guide wire in a dispenser case (not shown). Additionally, the aforementioned respective instruments that constitute the medical dilating instrument set 200 are included in a package or are individually provided.

In addition, the invention is not limited to the aforementioned embodiment, and forms, such as various modifications and improvements, will also be included in the invention as long as the object of the invention is achieved.

### <Second Embodiment>

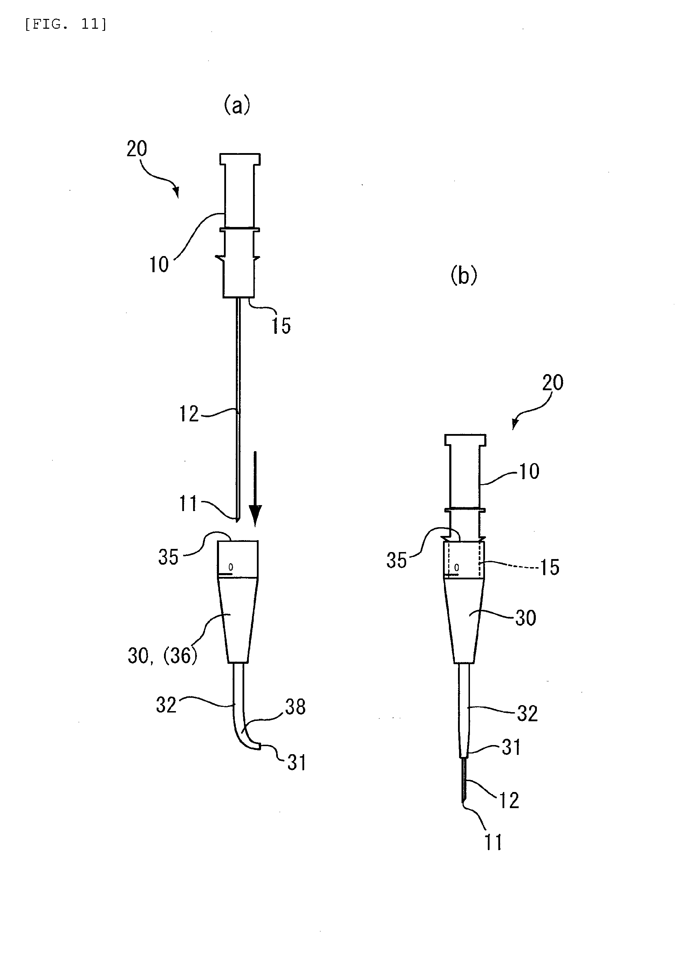

FIG. 11 (a) is an exploded front view of the cannular needle 20 of a second embodiment of the invention. Specifically, FIG. 11(a) is a front view showing a state where the needle body 10 is inserted into the guide portion 30 in a direction shown by an arrow. FIG. 11 (b) is a front view of the cannular needle 20 obtained by connecting the guide portion 30 and the needle body 10. FIG. 11(a) shows the protection state of the cannular needle 20, and FIG. 11 (b) shows the puncture state of the cannular needle 20.

The guide portion 30 of the present embodiment is different from that of the first embodiment in that there is a curved pipe portion 38. The curved pipe portion 38 is a tubular member having flexibility. The curved pipe portion 38 is made of soft resin materials, such as fluorine, urethane, and silicone rubber. The bending rigidity of the curved pipe portion 38 is sufficiently smaller than the bending rigidity of the needle portion 12. The curved pipe portion 38 and the other portions in the guide portion 30 may be made of the same materials, or the curved pipe portion 38 may be made of another materials with lower rigidity compared to the aforementioned other portions. The centerline of the curved pipe portion 38 is curved or crooked with respect to the centerline (the vertical direction of FIG. 11(a)) of the guide portion 30. The capillary portion 32 of the present embodiment has a linear shape as the first embodiment on the base end side, and a partial length thereof on the tip end side is curved. The tip end 31 of the capillary portion 32 is directed laterally with respect to the centerline of the guide portion 30, specifically, is directed to a direction substantially orthogonal to the centerline, in the natural state (protection state) shown in FIG. 11 (a). The position of the curved pipe portion 38 in the guide portion 30 is not particularly limited. The curved pipe portion 38 may be formed at a tip end portion including the tip end 31. Otherwise, a tip end portion including the tip end 31 may be formed in the shape of a straight pipe, and the curved pipe portion 38 may be formed at an intermediate portion of the guide portion 30.

The curved pipe portion 38 and the capillary portion 32 communicate with the inner cavity 36 of the diameter-enlarging portion 42. As shown in FIG. 11(a), the needle tip 11 of the needle portion 12 is inserted into the mounting portion 35 that is the opening of the guide portion 30 on the base end side. The needle tip 11 reaches the capillary portion 32 from the inner cavity 36. Moreover, if the needle tip 11 reaches the curved pipe portion 38, the needle tip 11 deforms the curved pipe portion 38 straightly (refer to FIG. 11(b)). The length of the capillary portion 32 including the curved pipe portion 38 is equal to the capillary portion 32 of the first embodiment, and the needle tip 11 protrudes further than the tip end 31 of the capillary portion 32 in the puncture state where the head 15 is mounted on the mounting portion 35.

As the tip end 31 of the capillary portion 32 in the natural state is directed laterally with respect to the centerline of the guide portion 30 as in the present embodiment, the curved pipe portion 38 is flexibly curved when the tip end 31 has bumped against the body cavity wall (the rear wall of the stomach) in the protection state where the needle body 10 is extracted from the guide portion 30. Accordingly, even when the insertion depth of the dilator 40 is large, the tip end 31 does not strongly contact the body cavity wall. The curved angle of the curved pipe portion 38, that is, the angle formed between the centerline of a proximal portion of the curved pipe portion 38 and the centerline of a distal portion is 15 degrees or more and 180 degrees or less and preferably 45 degrees or more and 120 degrees or less. By adopting the above angles, excessive load is not generated in the curved pipe portion 38 and the needle body 10 when the cannular needle 20 is configured, and the body cavity wall can be sufficiently protected when the dilator 40 is configured.

### <Third Embodiment>

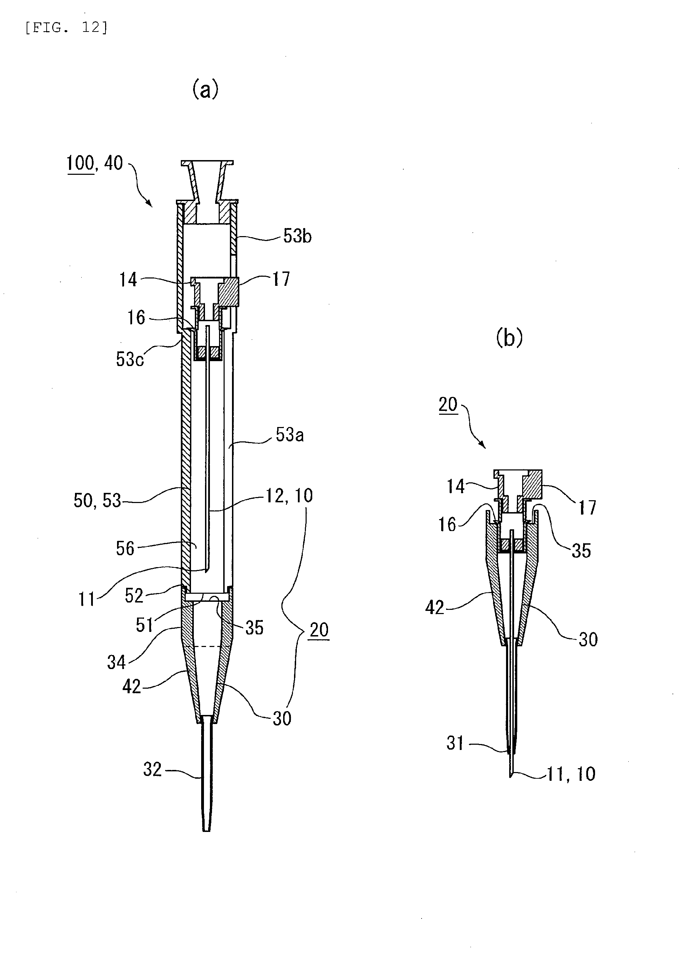

FIG. 12(a) is a longitudinal cross-sectional view showing the medical dilating instrument 100 of a third embodiment of the invention. This drawing shows the protection state of the cannular needle 20. Illustration of the sheath 60 (refer to FIG. 3) mounted around the trunk portion 50 will not be repeated. FIG. 12 (b) is a longitudinal cross-sectional view showing the puncture state of the cannular needle 20 of the present embodiment.

A hollow accommodating portion 56 is formed inside the trunk portion 50 of the present embodiment. The needle body 10 is different from those of the first and the second embodiments in that the needle body 10 is accommodated in the accommodating portion 56 in the protection state. In the medical dilating instrument 100 of the present embodiment, the tip end portion 52 of the trunk portion 50 is mounted on the base end portion 34 in a state where the head 15 of the needle body 10 is fitted to the base end portion 34 of the guide portion 30.

A sliding portion 17 is formed at the base end portion 14 of the needle body 10 so as to protrude laterally (to the right side of respective drawings of FIG. 12). The sliding portion 17 has an elongated shape that extends in the direction of the tip and base ends (the vertical direction of the respective drawings). A slit 53a is formed in the axial direction in the straight tube portion 53 of the trunk portion 50. The sliding portion 17 slides in the axial direction inside the slit 53a. A base end portion of the straight tube portion 53 is formed with an enlarged-diameter head 53b in which the internal diameter of the accommodating portion 56 increases in diameter. The slit 53a extends from an intermediate portion of the enlarged-diameter head 53b to a tip end 51. The needle body 10 has a smaller diameter than the internal diameter of the straight tube portion 53 of the trunk portion 50 except for the collar portion 16. The external diameter of the collar portion 16 is smaller than the internal diameter of the enlarged-diameter head 53b, and is larger than the internal diameter of the straight tube portion 53. The collar portion 16 is made of flexible materials, such as resin, is pressed against and deflected by an inner wall surface of the straight tube portion 53 inside the straight tube portion 53, and, is elastically restored to the natural state inside the enlarged-diameter head 53b. Operators, such as a doctor, can operate the sliding portion 17 to make the needle body 10 slide inside the guide portion 30 and the trunk portion 50.

The cannular needle 20 of the present embodiment freely shifts to the protection state and the puncture state. In the protection state shown in FIG. 12(a), the collar portion 16 of the needle body 10 is sealed by a step surface 53c of a lower end of the enlarged-diameter head 53b. In this protection state, the needle tip 11 of the needle body 10 is located further toward the base end side than the tip end 51 of the trunk portion 50. That is, the needle portion 12 of the needle body 10 is accommodated in the trunk portion 50 over its total length.

In the puncture state shown in FIG. 12(b), the collar portion 16 of the needle body 10 abuts against the mounting portion 35 of the guide portion 30 whereby the needle body 10 and the guide portion 30 are connected together. The needle tip 11 of the needle body 10 protrudes from the tip end 31 of the guide portion 30. Accordingly, the cannular needle 20 is configured.

In the medical dilating instrument 100 of the present embodiment, the trunk portion 50 is further mounted on the mounting portion 35 in a state where the needle body 10 is mounted on the mounting portion 35 of the guide portion 30. The mounting portion 35 of the guide portion 30 of the present embodiment has a step-like shape, and the tip end 51 of the trunk portion 50 is ruggedly fitted around the collar portion 16 mounted on the mounting portion 35 (refer to FIG. 12 (a)). Accordingly, in the fourth step of the gastric fistula formation shown in FIG. 8(a), it is possible to connect the tip end portion 52 of the trunk portion 50 with the mounting portion 35 of the guide portion 30 in a state where the needle body 10 is connected to the guide portion 30. By pulling the needle body 10 into the base end side of the trunk portion 50 as shown in FIG. 12 (a) after the dilator 40 is configured by connecting the trunk portion 50 to the guide portion 30 without extracting the needle body 10 from the guide portion 30 in this way, the medical dilating instrument 100 (cannular needle 20) can be shifted to the protection state. Accordingly, it is possible to safely discard the dilator 40 without touching of the needle tip 11 of the needle body 10 by an operator after the termination of the procedures of the gastric fistula forming method.

### <Fourth Embodiment>

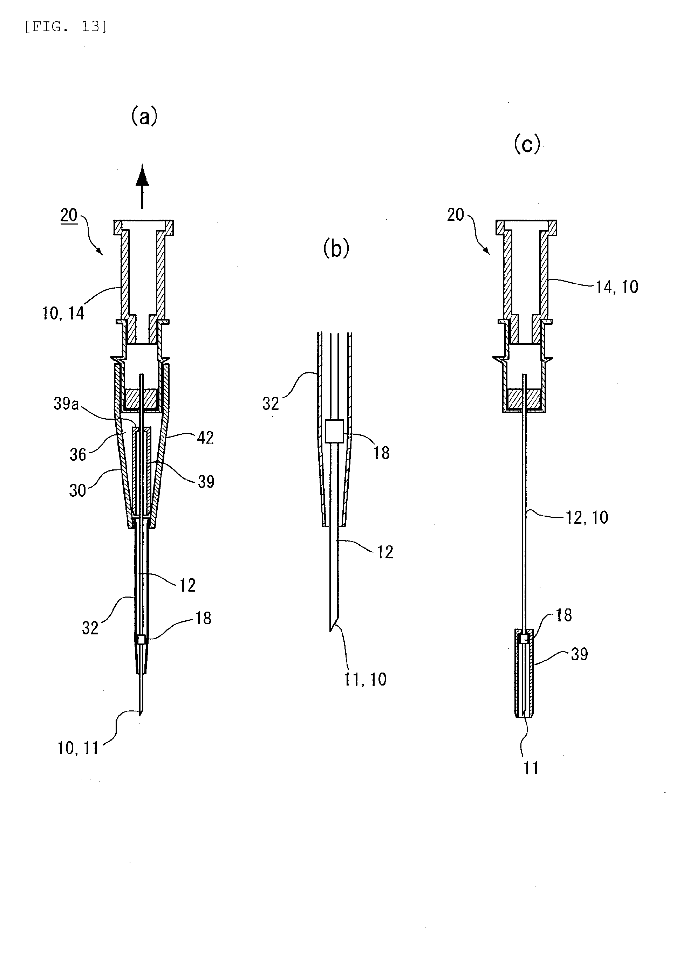

FIG. 13(a) is a longitudinal cross-sectional view showing the cannular needle 20 of a fourth embodiment of the invention. FIG. 13 (b) is an enlarged cross-sectional view of the vicinity of the needle tip 11. FIG. 13(c) is a longitudinal cross-sectional view showing the needle body 10 on which a needle tip protecting member 39 is mounted.

The needle tip protecting member 39 is mounted inside or around the guide portion 30 of the present embodiment (refer to FIG. 13 (a)). Also, there is a feature that the needle tip protecting member 39 is mounted on the needle body 10 to cover the needle tip 11 by extracting the needle body 10 in the puncture state from the guide portion 30 (refer to FIG. 13(c)).

When the needle body 10 is extracted from the cannular needle 20 after the second step (refer to FIG. 7(b)) of the gastric fistula formation, the needle tip protecting member 39 covers the needle tip 11 of the needle body 10. Accordingly, the needle body 10 can be safely discarded. Although the specific shape of the needle tip protecting member 39 is not particularly limited, the needle tip protecting member 39 of the present embodiment has a tube shape. The needle tip protecting member 39 opens on a tip end side, and has a top bottom 39a provided on a base end side.

The needle body 10 includes a protrusion 18 that is engaged with the needle tip protecting member 39 when being extracted from the guide portion 30. In the case of the present embodiment, the columnar protrusion 18 is anchored to an intermediate portion of the needle portion 12. The protrusion 18 is accommodated inside the capillary portion 32 in the puncture state.

If the needle body 10 is extracted in a direction shown by an arrow in FIG. 13(a) from the puncture state shown in this drawing, the protrusion 18 of the needle portion 12 reaches the diameter-enlarging portion 42 from the capillary portion 32. If the needle body 10 is further extracted, the protrusion 18 enters the inside of the needle tip protecting member 39 accommodated in the inner cavity 36 of the diameter-enlarging portion 42, and reaches the top bottom 39a of the needle tip protecting member 39. The top bottom 39a is formed with a sliding hole with a larger diameter than the external diameter of the needle portion 12 and a smaller diameter than the external diameter of the protrusion 18. Hence, if the needle body 10 is sufficiently extracted from the guide portion 30, the protrusion 18 of the needle body 10 abuts against the top bottom 39a, and is separated from the guide portion 30 while further pushing up the needle tip protecting member 39. FIG. 13 (c) shows this protection state.

### <Fifth Embodiment>

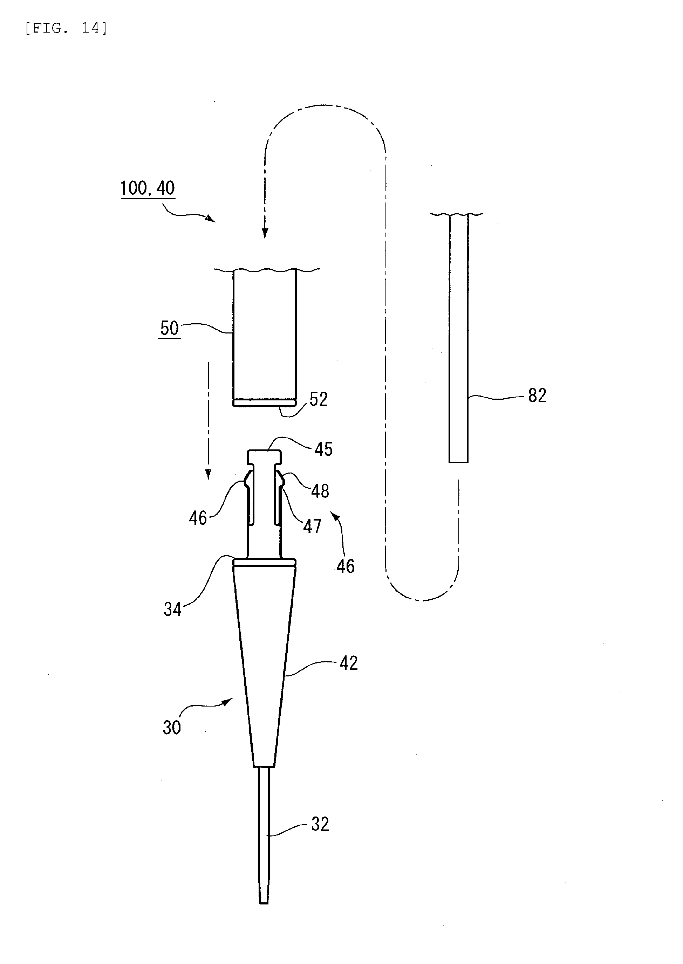

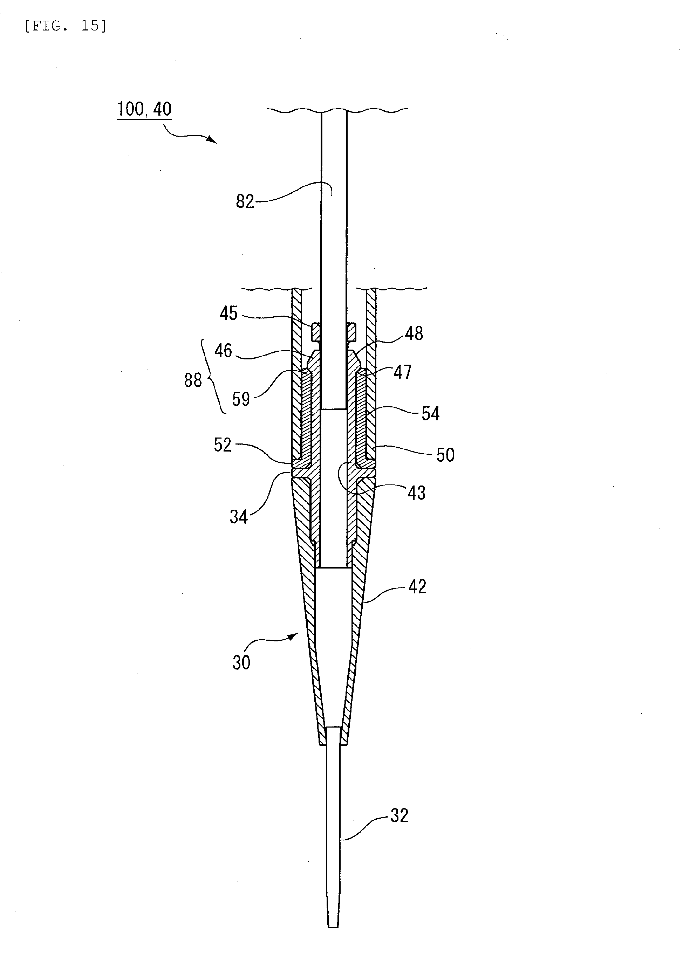

FIG. 14 is an exploded view of the medical dilating instrument 100 of a fifth embodiment of the invention. As for the trunk portion 50, only a portion on a tip end side is shown and illustration of a base end side will not be repeated. In FIG. 14, the tip end portion 52 of the trunk portion 50 is connected to the base end portion 34 of the guide portion 30, and a direction in which an engagement assisting member 82 is inserted into the trunk portion 50 is shown by an arrow. FIG. 15 is a cross-sectional schematic view of the medical dilating instrument 100 of the present embodiment. The dilator 40 is configured by connecting the cannular needle (that is, the guide portion 30), which is in the protection state by the needle body 10 (not shown) being extracted, and the trunk portion 50.

In the medical dilating instrument 100 of the present embodiment, similar to the first embodiment, the tip end portion 52 of the trunk portion 50 or the head 15 of the needle body 10 (refer to FIG. 1) is alternatively fitted to the base end portion 34 of the guide portion 30.

As shown in FIG. 15, the medical dilating instrument 100 of the present embodiment includes a locking mechanism 88 that releasably connects the guide portion 30 and the trunk portion 50. The locking mechanism 88 is a unit that prohibits the separation between the guide portion 30 and the trunk portion 50 that are in a mutually connected state.

More specifically, the locking mechanism 88 of the present embodiment includes a locking piece 46 and an engaging portion 59. The locking piece 46 is provided on one of the guide portion 30 or the trunk portion 50. The engaging portion 59 is provided on the other of the guide portion 30 or the trunk portion 50, and is engageable with the locking piece 46. By engaging the locking piece 46 and the engaging portion 59 on each other in a state where the guide portion 30 and the trunk portion 50 are connected to each other, the separation between the guide portion 30 and the trunk portion 50 is regulated. The phrase "the locking mechanism 88 releasably connects the guide portion 30 and the trunk portion 50" means that shifting is made to a first state where the guide portion 30 and the trunk portion 50 are directly or indirectly connected together and a second state where both are separable by the operation of the locking mechanism 88. Although the present embodiment shown in FIG. 15 illustrates that the guide portion 30 including the diameter-enlarging portion 42 is to be separated from the trunk portion 50, the invention is not limited to this. When the diameter-enlarging portion 42 is provided at the trunk portion 50, the diameter-enlarging portion 42 and the capillary portion 32 (guide portion 30) may be separated by the operation of the locking mechanism 88.

As shown in FIG. 15, a tubular connecting portion 43 is inserted into and fixed to an opening of the base end portion 34 of the diameter-enlarging portion 42. The diameter-enlarging portion 42 and the connecting portion 43 may be bonded together. A base end (an upper end of FIG. 15) of the connecting portion 43 is formed with an annular portion 45. The locking piece 46 is formed on the tip end side adjacent to the annular portion 45 of the connecting portion 43.

The locking piece 46 has a triangular claw shape that protrudes outward in the radial direction of the connecting portion 43. As shown in FIG. 15, the locking piece 46 is constituted by a lower taper 47 on the tip end (lower end) side, and an upper taper 48 on the base end (upper end) side. In addition, it is arbitrary to form the locking piece 46 with the upper taper 48.

A plurality of the locking pieces 46 are arranged around the connecting portion 43 so as to face each other or are arranged so as to be radially distributed. An individual locking piece 46 is able to be projected and retracted inward and outward in the radial direction of the connecting portion 43. The projection height of the locking piece 46 is increased by pressing the locking piece 46 outward from the inside of the tubular connecting portion 43.

A flange tube portion 54 is crowned on the tip end portion 52 of the trunk portion 50. A tubular upper end (engaging portion 59) of the flange tube portion 54 has a taper shape that inclines inward and downward. In addition, the flange tube portion 54 may be formed integrally with the trunk portion 50 from one material.

The medical dilating instrument 100 (dilator 40) of the present embodiment includes the engagement assisting member 82 (illustration of a portion thereof will not be repeated) used in a state being inserted into the trunk portion 50. As the locking piece 46 is biased outward in the radial direction by the engagement assisting member 82, the projection height of the locking piece 46 increases. Accordingly, as compared to a case where the engagement assisting member 82 is not used, the locking piece 46 is more deeply engaged with the engaging portion 59 by inserting the engagement assisting member 82 into the trunk portion 50. Accordingly, the lower taper 47 of the locking piece 46 and the engaging portion 59 are engaged with each other, and entry of the connecting portion 43 into the flange tube portion 54 is regulated. That is, the guide portion 30 and the trunk portion 50 are locked by inserting the engagement assisting member 82 into the trunk portion 50, and the guide portion 30 and the trunk portion 50 are unlocked by extracting the engagement assisting member 82. Both of the lower taper 47 and the engaging portion 59 have a taper shape that inclines inward and downward in the radial direction of the medical dilating instrument 100. In other words, the lower taper 47 and the engaging portion 59 incline in the same direction, and when the locking piece 46 is biased to the tip end side with respect to the engaging portion 59, the locking piece 46 is retracted inward in the radial direction of the medical dilating instrument 100 along this inclination direction. Accordingly, the external diameter of the locking piece 46 is deformed to be equal to or less than the internal diameter of the flange tube portion 54, and the locking piece 46 and the engaging portion 59 are separable from each other by some or all of the engagement therebetween being released. That is, the overall connecting portion 43 including the locking piece 46 is extractable from the flange tube portion 54 by extracting the engagement assisting member 82 from the trunk portion 50.

The engagement assisting member 82 has a rod shape. The external diameter of the engagement assisting member 82 is smaller than the internal diameter of the insertion hole 55 (refer to FIG. 2) of the trunk portion 50 and the internal diameter of the annular portion 45, and the engagement assisting member 82 is insertable into the insertion hole 55 and the annular portion 45. The dilator 40 of the present embodiment is not locked in a state where the engagement assisting member 82 is not inserted, and the guide portion 30 and the trunk portion 50 are engageable and releasable.

### <Sixth Embodiment>

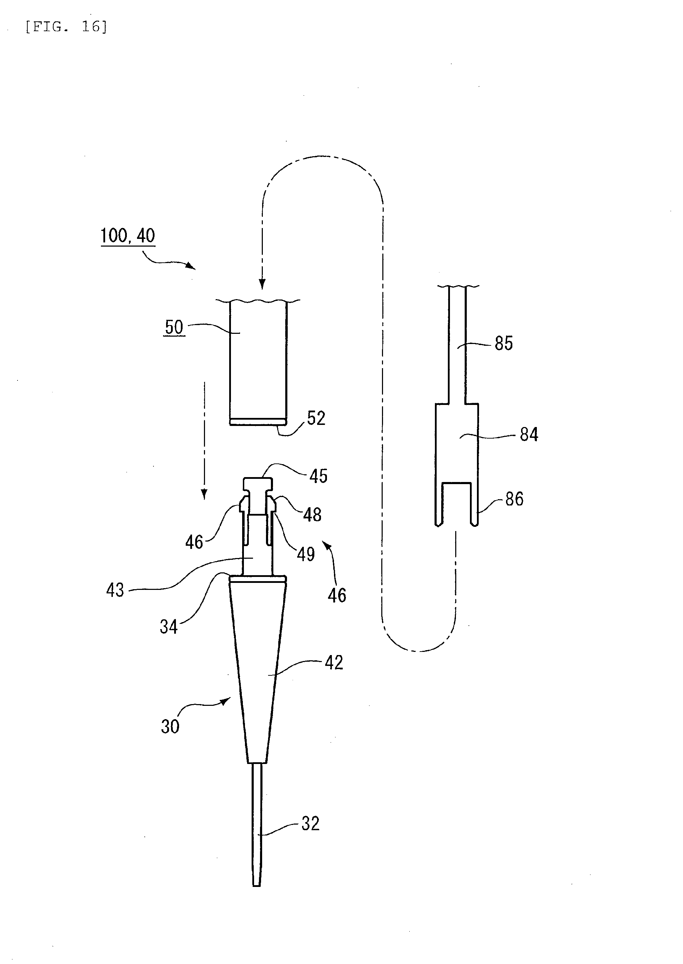

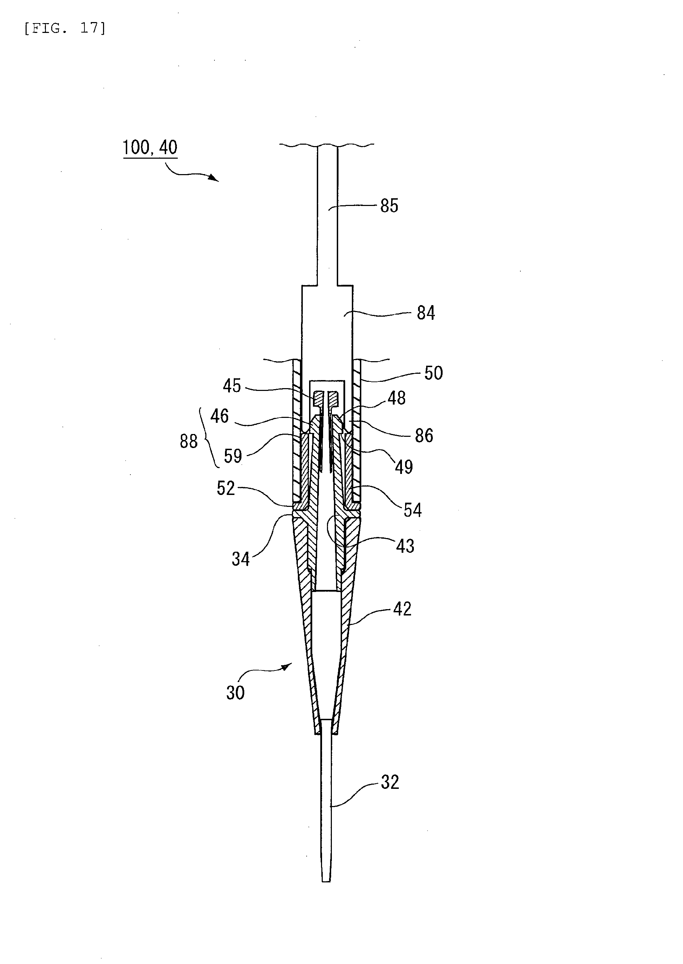

FIG. 16 is an exploded view of the medical dilating instrument 100 of a sixth embodiment of the invention. As for the trunk portion 50, only a portion on a tip end side is shown and illustration of a base end side will not be repeated. In FIG. 16, the tip end portion 52 of the trunk portion 50 is connected to the base end portion 34 of the guide portion 30, and a direction in which a releasing member 84 is inserted into the trunk portion 50 is shown by an arrow. FIG. 17 is a cross-sectional schematic view of the medical dilating instrument 100 of the present embodiment. The dilator 40 is configured by connecting the cannular needle (that is, the guide portion 30), which is in the protection state by the needle body 10 (not shown) being extracted, and the trunk portion 50.

The medical dilating instrument 100 of the present embodiment is common to that of the fifth embodiment in that this medical dilating instrument includes the locking mechanism 88 that releasably connects the guide portion 30 and the trunk portion 50. Also, the present embodiment is different from the fifth embodiment in that the guide portion 30 and the trunk portion 50 are locked in a non-insertion state of the releasing member 84 and are unlocked by inserting the releasing member 84 into the trunk portion 50.

That is, the medical dilating instrument 100 of the present embodiment includes the releasing member 84 used in a state being inserted into the trunk portion 50. Also, the locking piece 46 and the engaging portion 59 are separable from each other by inserting the releasing member 84 into the trunk portion 50 to release some or all of the engagement between the locking piece 46 and the engaging portion 59.

The locking piece 46 of the present embodiment is constituted by a stepped portion 49 on the tip end (lower end) side, and the upper taper 48 on the base end (upper end) side. The stepped portion 49, which is an end surface on a lower end side of the locking piece 46, is erected in the radial direction of the connecting portion 43.

As shown in FIG. 17, the upper end (engaging portion 59) of the flange tube portion 54 of the present embodiment is different from that of the fifth embodiment in that it is not in a taper shape that inclines inward and downward but rather, it is erected at a substantially right angle with respect to the axial direction of the flange tube portion 54. The external diameter of the locking piece 46 is larger than the internal diameter of the engaging portion 59. The locking piece 46 is able to be projected and retracted inward and outward in the radial direction of the tubular connecting portion 43. The projection height of the locking piece 46 is decreased by pressing the connecting portion 43 inward in the radial direction.

The releasing member 84 includes a rod-shaped shaft portion 85, and an annular pressing portion 86 formed at a tip end (a lower end) of the shaft portion 85. The pressing portion 86 has a larger diameter than the shaft portion 85. The external diameter of the pressing portion 86 is smaller than the internal diameter of the insertion hole 55 (refer to FIG. 2) of the trunk portion 50, and the releasing member 84 is insertable into the trunk portion 50. The internal diameter of the pressing portion 86 is smaller than the external diameter of the locking piece 46. As shown in FIG. 17, when the releasing member 84 is inserted into the trunk portion 50 in a state where the dilator 40 is configured by the guide portion 30 and the trunk portion 50 being connected, the diameter of the connecting portion 43 (locking piece 46) become a diameter such that the connection portion 43 is inserted into the flange tube portion 54 as the pressing portion 86 causes the locking piece 46 to be retracted inward in the radial direction. Accordingly, the locking mechanism 88 is unlocked and the guide portion 30 and the trunk portion 50 are separable from each other.

According to the medical dilating instruments 100 of the fifth and sixth embodiments, since the trunk portion 50 and the guide portion 30 are inseparably connected together by the locking mechanism 88, the diameter-enlarging portion 42 does not become unstable during dilating procedures. An operator is able to extract the engagement assisting member 82 from the trunk portion 50 of the dilator 40 or insert the releasing member 84 into the trunk portion 50 to unlock the locking mechanism 88, thereby simply separating the trunk portion 50 and the guide portion 30 from each other. Accordingly, for example, as shown in FIG. 8(b), even after the dilator 40 is configured, it is possible to easily separate the trunk portion 50 to finely operate the guide portion 30 or the guide wire 90 for a procedure.

The fifth and sixth embodiments illustrate that the locking mechanism 88 is operated for release, using the engagement assisting member 82 or the releasing member 84 that is a body separate from the guide portion 30 or the trunk portion 50. However, the invention is not limited to this. For example, as a first modification example of the sixth embodiment, a configuration may be adopted in which the engagement between the locking piece 46 and the engaging portion 59 is releasable by a finger operation. Specifically, slits through which the locking piece 46 in the state of being engaged with the engaging portion 59 is inserted may be formed in_a peripheral surface of the trunk portion 50. By thrusting the locking pieces 46 facing the slits into the trunk portion 50 with fingers, the engagement between the locking piece 46 and the engaging portion 59 are released and the connecting portion 43 becomes separable from the flange tube portion 54.

In order for the trunk portion 50 and the guide portion 30 to be reliably separated when the release operation of the locking mechanism 88 is performed, an elastic member (not shown) that biases the trunk portion 50 and the guide portion 30 in mutually separating directions may be provided. Specifically, as a second modification example of the sixth embodiment, an elastic member (spiral spring: not shown) may be provided inside the annular pressing portion 86. When the releasing member 84 is inserted into the trunk portion 50 and a lower end of the pressing portion 86 presses the locking piece 46 inward in the radial direction, this spiral spring is brought into a state where the spiral spring is compressed more than a natural length. Accordingly, when the engagement between the locking pieces 46 and the engaging portion 59 are released, this spiral spring elastically biases an upper end of the annular portion 45 or the upper taper 48 downward, and extraction of the connecting portion 43 from the flange tube portion 54 is assisted in.

According to the cannular needles 20 of the aforementioned respective embodiments, it is possible to safely discard the needle body 10 presented for the procedures of the gastric fistula forming method.

The aforementioned embodiments include the following technical ideas.
(1) A medical dilating instrument including a needle body having a pointed needle tip; an elongated guide portion formed so that a tip end thereof is duller than the needle tip; a trunk portion having a larger diameter compared to the guide portion; and a diameter-enlarging portion that is provided at the guide portion or the trunk portion and increases in diameter from a tip end side toward a base end side, wherein the needle body and the guide portion are shifted from a puncture state where the needle body and the guide portion are connected to each other, the needle body is arranged along the guide portion within the proximity, and the needle tip protrudes further than the tip end of the guide portion, to a protection state where the needle tip is retreated from the tip end of the guide portion, and wherein the trunk portion is connected to a base end portion of the guide portion in the protection state;
(2) The medical dilating instrument described in the above (1), wherein a base end portion of the needle body is formed with a head, and wherein a tip end portion of the trunk portion or the head of the needle body is alternatively fitted to the base end portion of the guide portion;
(3) The medical dilating instrument described in the above (1) or (2), wherein the guide portion has a tube shape including an inner cavity with a larger diameter than the needle body;
(4) The medical dilating instrument described in the above (3), wherein a valve body that permits insertion of the needle body and regulates an air current that flows from the tip end of the guide portion to the base end portion of the guide portion in the protection state is provided in the inner cavity of the guide portion;
(5) The medical dilating instrument described in any one of the above (1) to (4), further including a sheath that is used in a state being mounted around the trunk portion and is capable of being torn off or separable along an axis direction of the trunk portion;
(6) The medical dilating instrument described in the above (5), wherein the diameter-enlarging portion is provided at the guide portion, and the sheath covers a boundary between the guide portion and the trunk portion;
(7) The medical dilating instrument described in any one of the above (1) to (6), wherein a tip end of the guide portion is a curved pipe portion having flexibility;
(8) The medical dilating instrument according to any one of the above (1) to (7), wherein a hollow accommodating portion is formed inside the trunk portion, and the needle body is accommodated in the accommodating portion in the protection state;
(9) The medical dilating instrument described in any one of the above (1) to (7), wherein the guide portion is equipped with a needle tip protecting member, and the needle body in the puncture state is extracted from the guide portion and thereby the needle tip protecting member is mounted on the needle body to cover the needle tip.
(10) A medical dilating instrument set including a needle body having a pointed needle tip; a guide portion having an elongated shape formed so that a tip end thereof is duller than the needle tip and having a mounting portion at a base end; a trunk portion having a larger diameter than the guide portion; and a diameter-enlarging portion that is provided at the guide portion or the trunk portion and increases in diameter from a tip end side toward a base end side, wherein the needle body, the guide portion, and the trunk portion are constituted as separate bodies, wherein the needle body or the trunk portion is mounted on the mounting portion and thereby the guide portion is arranged along the needle body within the proximity by mounting the needle body on the mounting portion and the needle tip protrudes further than the tip end of the guide portion, and wherein the trunk portion is mounted on the mounting portion from which the needle body is separated;
(11) The medical dilating instrument set described in the above (10), further including a sheath that is used in a state being mounted around the trunk portion and is capable of being torn off along an axis direction of the trunk portion; and
(12) The medical dilating instrument set described in the above (11), further including a gastrostomy catheter having a flexible intracorporeal indwelling portion formed at a tip; and an obturator that extends the intracorporeal indwelling portion to thereby reduce the diameter of the intracorporeal indwelling portion to a fine diameter smaller than the sheath.

The aforementioned embodiments further include the following technical ideas.
(i) The medical dilating instrument described in any one of the above (1) to (7), further including a locking mechanism that releasably connects the guide portion and the trunk portion;
(ii) The medical dilating instrument described in the above (i), wherein the locking mechanism includes a locking piece provided on one of the guide portion and the trunk portion, and an engaging portion provided on the other of the guide portion and the trunk portion and engageable with the locking piece, and wherein the separation between the guide portion and the trunk portion is regulated by engaging the locking piece and the engaging portion with each other in a state where the guide portion and the trunk portion are connected to each other;
(iii) The medical dilating instrument described in the above (ii), further including an engagement assisting member used in a state being inserted into the trunk portion, wherein the locking piece is more deeply engaged with the engaging portion by inserting the engagement assisting member into the trunk portion, and some or all of the engagement is released by extracting the engagement assisting member from the trunk portion so that the locking piece and the engaging portion are separable from each other; and
(iv) The medical dilating instrument described in the above (ii), further including a releasing member used in a state being inserted into the trunk portion, wherein some or all of the engagement between the locking piece and the engaging portion is released by inserting the releasing member into the trunk portion so that the locking piece and the engaging portion are separable from each other.

This application claims priority based on Japanese Patent Application No. 2011-239229, filed on October 31, 2011, the disclosure of which is all incorporated herein by reference.

## Claims

1. A medical dilating instrument comprising:
a needle body having a pointed needle tip;
an elongated guide portion formed so that a tip end thereof is duller than the needle tip;
a trunk portion having a larger diameter compared to the guide portion; and
a diameter-enlarging portion that is provided at the guide portion or the trunk portion and increases in diameter from a tip end side toward a base end side,
wherein the needle body and the guide portion are shifted from a puncture state where the needle body and the guide portion are connected to each other, the needle body is arranged along the guide portion within the proximity, and the needle tip protrudes further than the tip end of the guide portion, to a protection state where the needle tip is retreated from the tip end of the guide portion, and
wherein the trunk portion is connected to a base end portion of the guide portion in the protection state.

2. The medical dilating instrument according to claim 1,
wherein a base end portion of the needle body is formed with a head, and
wherein a tip end portion of the trunk portion or the head of the needle body is alternatively fitted to the base end portion of the guide portion.

3. The medical dilating instrument according to claim 1 or 2,
wherein the guide portion has a tube shape including an inner cavity with a larger diameter compared to the needle body.

4. The medical dilating instrument according to claim 3,
wherein a valve body that permits insertion of the needle body and regulates an air current that flows from the tip end of the guide portion to the base end portion of the guide portion in the protection state is provided in the inner cavity of the guide portion.

5. The medical dilating instrument according to any one of claims 1 to 4, further comprising:
a sheath that is used in a state being mounted around the trunk portion and is capable of being torn off or separable along an axis direction of the trunk portion.

6. The medical dilating instrument according to claim 5,
wherein the diameter-enlarging portion is provided at the guide portion, and the sheath covers a boundary between the guide portion and the trunk portion.

7. The medical dilating instrument according to any one of claims 1 to 6,
wherein the guide portion has a curved pipe portion.

8. The medical dilating instrument according to any one of claims 1 to 7, further comprising:
a locking mechanism that releasably connects the guide portion and the trunk portion.

9. The medical dilating instrument according to claim 8,
wherein the locking mechanism includes a locking piece provided on one of the guide portion and the trunk portion, and an engaging portion provided on the other of the guide portion and the trunk portion and engageable with the locking piece, and
wherein the separation between the guide portion and the trunk portion is regulated by engaging the locking piece and the engaging portion with each other in a state where the guide portion and the trunk portion are connected to each other.

10. The medical dilating instrument according to claim 9, further comprising:
an engagement assisting member used in a state being inserted into the trunk portion,
wherein the locking piece is more deeply engaged with the engaging portion by inserting the engagement assisting member into the trunk portion, and some or all of the engagement is released by extracting the engagement assisting member from the trunk portion so that the locking piece and the engaging portion are separable from each other.

11. The medical dilating instrument according to claim 9, further comprising:
a releasing member used in a state being inserted into the trunk portion,
wherein some or all of the engagement between the locking piece and the engaging portion is released by inserting the releasing member into the trunk portion so that the locking piece and the engaging portion are separable from each other.

12. The medical dilating instrument according to any one of claims 1 to 11,
wherein a hollow accommodating portion is formed inside the trunk portion, and the needle body is accommodated in the accommodating portion in the protection state.

13. The medical dilating instrument according to any one of claims 1 to 11,
wherein the guide portion is equipped with a needle tip protecting member, and the needle body in the puncture state is extracted from the guide portion and thereby the needle tip protecting member is mounted on the needle body to cover the needle tip.

14. A medical dilating instrument set comprising:
a needle body having a pointed needle tip;
a guide portion having an elongated shape formed so that a tip end thereof is duller than the needle tip and having a mounting portion at a base end;
a trunk portion having a larger diameter than the guide portion; and
a diameter-enlarging portion that is provided at the guide portion or the trunk portion and increases in diameter from a tip end side toward a base end side,
wherein the needle body, the guide portion, and the trunk portion are constituted as separate bodies,
wherein the needle body or the trunk portion is mounted on the mounting portion and thereby the guide portion is arranged along the needle body within the proximity by mounting the needle body on the mounting portion and the needle tip protrudes further than the tip end of the guide portion, and
wherein the trunk portion is mounted on the mounting portion from which the needle body is separated.

15. The medical dilating instrument set according to claim 14, further comprising:
a sheath that is used in a state being mounted around the trunk portion and is capable of being torn off along an axis direction of the trunk portion.

16. The medical dilating instrument set according to claim 15, further comprising:
a gastrostomy catheter having a flexible intracorporeal indwelling portion formed at a tip; and
an obturator that extends the intracorporeal indwelling portion to thereby reduce the diameter of the intracorporeal indwelling portion to a fine diameter smaller than the sheath.
